(19) Europäisches Patentamt
European Patent Office
Office européen des brevets

(11) EP 0 509 433 B1

(12) **EUROPÄISCHE PATENTSCHRIFT**

(45) Veröffentlichungstag und Bekanntmachung des Hinweises auf die Patenterteilung:
**19.07.2000 Patentblatt 2000/29**

(51) Int. Cl.$^7$: **C07D 261/04**, C07D 275/02, A01N 25/32

(21) Anmeldenummer: **92106365.7**

(22) Anmeldetag: **13.04.1992**

(54) **Isoxazoline oder Isothiazoline enthaltende pflanzenschützende Mittel, neue Isoxazoline und Isothiazoline und Verfahren zu deren Herstellung**

Plant protecting agents containing isoxazolines or isothiazolines, new isothiazolines and isoxazolines and process for their preparation

Agents phytosanitaires contenant des isoxazolines ou des isothiazolines, nouvelles isoxazolines et isothiazolines et procédé de préparation

(84) Benannte Vertragsstaaten:
**DE DK ES FR GB IT NL**

(30) Priorität: **15.04.1991 DE 4112251**

(43) Veröffentlichungstag der Anmeldung:
**21.10.1992 Patentblatt 1992/43**

(73) Patentinhaber:
**Aventis CropScience GmbH**
**13509 Berlin (DE)**

(72) Erfinder:
- **Löher, Heinz-Josef, Dr.**
  **W-6237 Liederbach (DE)**
- **Bauer, Klaus, Dr.**
  **W-6450 Hanau (DE)**
- **Bieringer, Hermann, Dr.**
  **W-6239 Eppstein/Taunus (DE)**

(56) Entgegenhaltungen:
**WO-A-91/08202** **DE-A- 4 026 018**
**US-A- 4 889 551**

- **JOURNAL OF ORGANIC CHEMISTRY. Bd. 25, Nr. 7, 8. Juli 1960, EASTON US Seiten 1160 - 1164; WYMAN R. VAUGHAN ET AL: '5-Phenyl-2-isoxazoline-3-carboxylic acid'**
- **SYNTHESIS. Nr. 6, Juni 1986, STUTTGART DE Seiten 488 - 490; TOMIO SHIMIZU ET AL: 'Synthesis of isoxazoline-3-carboxanilides and isoxazole-3-carboxanilides by thermolysis of alpha-methoxycarbonyl-alpha nitroacetanilides in the presence of dipolarophiles'**
- **TETRAHEDRON Bd. 41, Nr. 4, 1985, OXFORD GB Seiten 727 - 738; TOMIO SHIMIZU ET AL: 'Reaction of 3,4-disubstituted 1,2,5-oxadiazole 2-oxides with dipolarophiles'**

Bemerkungen:
Die Akte enthält technische Angaben, die nach dem Eingang der Anmeldung eingereicht wurden und die nicht in dieser Patentschrift enthalten sind.

EP 0 509 433 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.7 (HRS)/3.6

**Beschreibung**

Bei der Anwendung von Herbiziden können unerwünschte, nicht tolerierbare Schäden an Kulturpflanzen auftreten. Besonders bei der Applikation von Herbiziden nach dem Auflaufen der Kulturpflanzen besteht daher oft das Bedürftnis, das Risiko einer möglichen Phytotoxizität zu vermeiden.

Solche Verbindungen, welche die Eigenschaft besitzen, Kulturpflanzen gegen phytotoxische Schäden durch Herbizide zu schützen, ohne die eigentliche herbizide Wirkung dieser Mittel zu beeinträchtigen, werden "Antidot" oder "Safener" genannt.

Verschiedene Verbindungen wurden für diese Anwendung bereits beschrieben (vgl. z.B. EP-A-152 006 oder EP-A-174 562). Die Anwendung bestimmter Isoxazoline und Isothiazoline als Safener wurde in der deutschen Patentanmeldung P 40 26 018.6 (HOE 90/F 251) vorgeschlagen. Aus US-A-4,889,551 sind außerdem strukturisomere 3-Phenyl-2-isoxazolin-5-carbonsäurederivate als Pflanzenwachstumsregulatoren bekannt.

Die Erfindung betrifft kulturpflanzenschützende Mittel, welche Isoxazoline oder Isothiazoline der allgemeinen Formel I oder deren Salze,

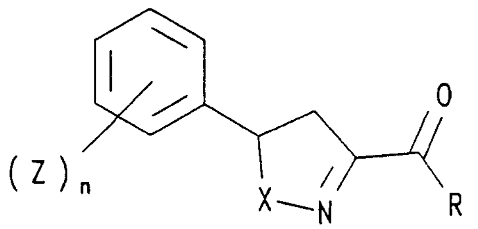

(I)

in welcher

X ein Sauerstoff oder Schwefelatom, insbesondere ein Sauerstoffatom bedeutet,

R

a) Hydroxy, Mercapto, $(C_1-C_5)$Alkoxy, $(C_2-C_5)$Alkenyloxy, $(C_2-C_5)$Alkinyloxy, $(C_1-C_5)$Alkylmercapto, $(C_2-C_5)$Alkenylmercapto, $(C_2-C_5)$Alkinylmercapto, $(C_3-C_7)$Cycloalkyloxy oder $(C_3-C_7)$Cycloalkylmercapto bedeutet, wobei die 8 letztgenannten Gruppen unsubstituiert oder mit einem oder mehreren, vorzugsweise bis zu drei gleichen oder verschiedenen Resten aus der Reihe $(C_6-C_{12})$Aryl, $(C_1-C_5)$Alkoxy, $(C_2-C_5)$Alkenyloxy, $(C_2-C_5)$Alkinyloxy, $(C_6-C_{12})$Aralkyloxy, $(C_6-C_{12})$Aryloxy, $(C_3-C_7)$Cycloalkyloxy, $(C_1-C_5)$Alkylmercapto, Mono- oder Di-$(C_1-C_5)$alkylamino, Cyano, Halogen und Nitro substituiert sind,

b) $(C_6-C_{12})$Aralkyloxy, $(C_6-C_{12})$Aryloxy, $(C_6-C_{12})$Aralkylmercapto oder $(C_6-C_{12})$Arylmercapto, welche jeweils unsubstituiert oder mit einem oder mehreren, vorzugsweise bis zu fünf gleichen oder verschiedenen Resten aus der Reihe $(C_1-C_5)$Alkyl, $(C_2-C_5)$Alkenyl, $(C_2-C_5)$Alkinyl, Halogen, Cyano, Nitro, $(C_1-C_5)$Alkoxy, $(C_2-C_5)$Alkenyloxy, $(C_2-C_5)$Alkinyloxy, $(C_1-C_5)$Alkylmercapto, Mono- oder Di-$(C_1-C_5)$alkylamino, $(C_6-C_{12})$Aryloxy und $(C_6-C_{12})$Aroyloxy substituiert sind, oder Tri-$(C_1-C_5)$alkylsilyl-$(C_1-C_5)$alkoxy, $(C_6-C_{12})$Aryldi-$(C_1-C_5)$alkylsilyloxy, $(C_6-C_{12})$Aryl$(C_1-C_5)$alkyldi-$(C_1-C_5)$alkylsilyloxy, Di-$(C_6-C_{12})$aryl$(C_1-C_5)$alkylsilyloxy oder Di-$[(C_6-C_{12})$aryl$(C_1-C_5)$alkyl$]$-$(C_1-C_5)$alkylsilyloxy bedeutet,

c) einen Rest der Formel NR'R', worin die R' für gleiche oder verschiedene Reste aus der Gruppe Wasserstoff, $(C_1-C_5)$Alkyl, $(C_2-C_5)$Alkenyl, $(C_2-C_5)$Alkinyl und $(C_3-C_7)$Cycloalkyl steht, oder Pyridino, Morpholino, Di-$(C_1-C_5)$alkylmorpholino, Hydrazino oder einen Rest der Formel

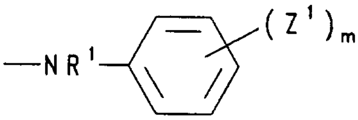

bedeutet,

worin $R^1$ Wasserstoff, $(C_1$-$C_5)$Alkyl, $(C_2$-$C_5)$Alkenyl oder $(C_2$-$C_5)$Alkinyl, die Reste $Z^1$ unabhängig voneinander Halogen, Nitro, $(C_1$-$C_5)$Alkyl, $(C_2$-$C_5)$Alkenyl, $(C_1$-$C_5)$Alkoxy oder $(C_6$-$C_{12})$Aryloxy und m eine ganze Zahl von 0 bis 5 sind,

d) einen Rest der Formel

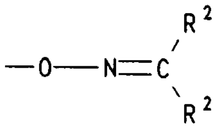

bedeutet, worin die Reste $R^2$ unabhängig voneinander $(C_1$-$C_5)$Alkyl oder gemeinsam mit dem sie verknüpfenden C-Atom $(C_3$-$C_7)$Cycloalkyliden bedeuten,

e) einen Rest der Formel $-O-CR^3R^3-CO-R^4$ bedeutet, worin $R^3$ für gleiche oder verschiedene Reste aus der Reihe Wasserstoff, $(C_1$-$C_5)$Alkyl, $(C_2$-$C_5)$Alkenyl, $(C_2$-$C_5)$Alkinyl, $(C_6$-$C_{12})$Aryl, $(C_6$-$C_{12})$Aryl $(C_1$-$C_5)$alkyl, $(C_1$-$C_5)$Alkoxy, $(C_2$-$C_5)$Alkenyloxy, $(C_2$-$C_5)$Alkinyloxy und $(C_6$-$C_{12})$Aryloxy steht und $R^4$ für Wasserstoff, $(C_1$-$C_5)$Alkyl, $(C_2$-$C_5)$Alkenyl, $(C_2$-$C_5)$Alkinyl, $(C_6$-$C_{12})$Aryl oder $(C_6$-$C_{12})$Aryl-$(C_1$-$C_5)$alkyl steht,

f) einen Rest der Formel

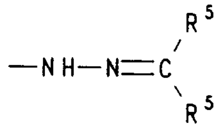

bedeutet, worin $R^5$ für gleiche oder verschiedene Reste aus der Reihe Wasserstoff, $(C_1$-$C_5)$Alkyl und $(C_6$-$C_{12})$Aryl steht oder die beiden Reste $R^5$ gemeinsam mit dem sie verknüpfenden C-Atom $(C_3$-$C_7)$Cycloalkyliden bedeuten, oder

g) einen Rest der Formel $-O-CR^6R^6-CO-R^7$ bedeutet, worin $R^6$ für gleiche oder verschiedene Reste aus der Reihe Wasserstoff, $(C_1$-$C_5)$Alkyl, $(C_2$-$C_5)$Alkenyl, $(C_2$-$C_5)$Alkinyl, $(C_6$-$C_{12})$Aryl, $(C_{6-12})$Aryl-$(C_1$-$C_5)$alkyl, $(C_1$-$C_5)$Alkoxy, $(C_2$-$C_5)$Alkenyloxy, $(C_2$-$C_5)$Alkinyloxy und $(C_6$-$C_{12})$Aryloxy steht und $R^7$ eine der vorstehend für R unter a) bis f) angegebenen Bedeutungen hat,

Z Halogen, Nitro, Cyano, $(C_1$-$C_4)$-Alkyl, $(C_1$-$C_4)$-Alkoxy, $(C_1$-$C_4)$-Alkylmercapto, wobei die Alkyl-, Alkoxy- und Alkylmercaptogruppen unabhängig voneinander jeweils unsubstituiert oder mit einem oder mehreren, vorzugsweise bis zu sechs Halogenatomen, insbesondere Fluor oder Chlor, substituiert sind, $(C_3$-$C_6)$-Cycloalkyl, das unsubstituiert oder mit vorzugsweise bis zu drei $(C_1$-$C_4)$-Alkyl substituiert ist, Amino, Hydroxymethyl, $(C_1$-$C_4)$-Alkylamino, Di-$(C_1$-$C_4)$-alkylamino, $(C_1$-$C_4)$-Alkoxymethyl, wobei die Alkyl- und Alkoxygruppen in den drei letztgenannten Resten unabhängig voneinander unsubstituiert oder mit vorzugsweise bis zu drei $(C_1$-$C_4)$-Alkyl substituiert sind, $(C_6$-$C_{12})$Aryl oder $(C_6$-$C_{12})$Aryloxy, wobei Aryl und Aryloxy unabhängig voneinander jeweils unsubstituiert oder ein- oder mehrfach, vorzugsweise mit bis zu fünf gleichen oder verschiedenen Resten aus der Reihe Halogen und Trifluormethyl substituiert sind, bedeutet und

n eine ganze Zahl von 0 bis 5, insbesondere 0 bis 3 ist,

und übliche Formulierungshilfsmittel enthalten.

Die Erfindung betrifft selektive herbizide Mittel, die einen Wirkstoff der genannten Formel I oder deren Salze in Kombination mit einem Herbizid und gegebenenfalls übliche Formulierungshilfsmittel enthalten.

In der Formel I können die Alkyl-, Alkenyl- und Alkinylreste jeweils geradkettig oder verzweigt sein. Sie haben vorzugsweise bis zu fünf C-Atome. Entsprechendes gilt für die davon abgeleiteten Reste, wie Alkoxy, Alkylmercapto, Alkylamino, Dialkylamino und die entsprechenden ungesättigten Reste.

Cycloalkylreste und davon abgeleitete Reste wie Cycloalkyloxy oder Cycloalkylmercapto haben vorzugsweise 3 bis 7 C-Atome.

Arylreste haben vorzugsweise 6 bis 12 C-Atome; bevorzugt sind Phenyl, Naphthyl und Biphenylyl, insbesondere Phenyl. Entsprechendes gilt für davon abgeleitete Reste, wie Aryloxy, Arylmercapto, Aroyl, Aralkyl, Aralkyloxy und Aralkylmercapto. Aralkyl ist vorzugsweise Benzyl.

Halogen bedeutet Fluor, Chlor, Brom oder Iod, vorzugsweise Fluor, Chlor oder Brom, insbesondere Fluor oder Chlor.

Im Falle R = Hydroxy können die Verbindungen der Formel I Salze bilden. Erfindungsgemäß einsetzen lassen sich die in der Landwirtschaft verwendbaren Salze. Als solche kommen beispielsweise Metallsalze wie Alkali- oder Erdalkalisalze, insbesondere Natrium- oder Kaliumsalze, Salze mit Ammonium, Mono-, Di-, Tri- oder Tetra-($C_1$-$C_4$)-alkylammonium oder mit Mono-, Di-, Tri-oder Tetra-($C_1$-$C_4$)-alkanolammonium in Frage.

Die Erfindung betrifft insbesondere auch alle Stereoisomeren und deren Gemische, die von der Formel I umfaßt, jedoch nicht spezifisch definiert sind. Stereoisomere können vor allem auftreten, wenn ein oder mehrere asymmetrische C-Atome und/oder geeignet substituierte Doppelbindungen in den Verbindungen der Formel I vorhanden sind. Die Stereoisomere lassen sich aus racemischen Gemischen nach üblichen Trennmethoden erhalten. Alternativ können Stereoisomere durch Einsatz stereoselektiver Reaktionen unter Verwendung optisch aktiver Ausgangs- oder Hilfsstoffe selektiv hergestellt werden.

Von besonderem Interesse sind erfindungsgemäße pflanzenschützende oder selektive herbizide Mittel, die eine Verbindung der genannten Formel I enthalten, worin

R

a) Hydroxy, Mercapto, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, ($C_2$-$C_4$)-Alkenylmercapto, ($C_2$-$C_4$)-Alkinylmercapto oder ($C_3$-$C_8$)-Cycloalkylmercapto bedeutet, wobei die 8 letztgenannten Gruppen unsubstituiert oder mit einem oder mehreren, vorzugsweise bis zu drei gleichen oder verschiedenen Resten aus der Reihe Aryl, ($C_1$-$C_4$)-Alkoxy, ($C_2$-$C_4$)-Alkenyloxy, ($C_2$-$C_4$)-Alkinyloxy, Aralkyloxy, Aryloxy, ($C_3$-$C_8$)-Cycloalkyloxy, ($C_1$-$C_4$)-Alkylmercapto, Mono-oder Di-($C_1$-$C_4$)-Alkylamino, Cyano, Halogen und Nitro substituiert sind,

b) Aryloxy, Arylmercapto, Aralkyloxy oder Aralkylmercapto, welche jeweils unsubstituiert oder mit einem oder mehreren, vorzugsweise bis zu fünf gleichen oder verschiedenen Resten aus der Reihe ($C_1$-$C_4$)-Alkyl, ($C_2$-$C_4$)-Alkenyl, ($C_2$-$C_4$)-Alkinyl, Halogen, Cyano, Nitro, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, Mono- oder Di-($C_1$-$C_4$)-Alkylamino, Aryloxy oder Aralkyloxy substituiert sind, oder Tri-($C_1$-$C_4$)-alkylsilylalkoxy bedeutet,

c) einen Rest der Formel -NR'R', worin R' Wasserstoff und/oder ($C_1$-$C_4$)-Alkyl bedeutet, Pyridino, Morpholino, Dimethylmorpholino, Hydrazino oder einen Rest der Formel

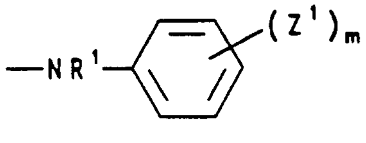

bedeutet, worin $R^1$ Wasserstoff oder ($C_1$-$C_4$)-Alkyl, die Reste $Z^1$ unabhängig voneinander Halogen, Nitro, ($C_1$-$C_4$)-Alkyl, ($C_1$-$C_4$)-Alkoxy oder Aryloxy und m 0 bis 3 sind,

d) einen Rest der Formel -O-N=$CR^2R^2$ bedeutet, worin $R^2$ für ($C_1$-$C_4$)-Alkyl, insbesondere Methyl steht oder die Reste $R^2$ gemeinsam mit dem sie verknüpfenden C-Atom für Cyclohexyliden oder Cyclopentyliden stehen,

e) einen Rest der Formel -O-$CR^3R^3$-CO-$R^4$, worin $R^3$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Aryl, Aralkyl oder ($C_1$-$C_4$)-Alkoxy steht und $R^4$ für ($C_1$-$C_4$)-Alkyl, ($C_2$-$C_4$)-Alkenyl, ($C_2$-$C_4$)-Alkinyl, Aryl oder Aralkyl steht,

f) einen Rest der Formel -NH-N=$CR^5R^5$, worin $R^5$ für gleiche oder verschiedene Reste aus der Reihe Wasserstoff, ($C_1$-$C_4$)-Alkyl oder Aryl oder die beiden Reste $R^5$ gemeinsam mit dem sie verknüpfenden C-Atom Cyclohexyliden oder Cyclopentyliden bedeuten, oder

g) einen Rest der Formel -O-$CR^6R^6$-CO-$R^7$ bedeutet, worin $R^6$ für gleiche oder verschiedene Reste aus der

Reihe Wasserstoff, $(C_1-C_4)$Alkyl, $(C_2-C_4)$-Alkenyl, $(C_2-C_4)$-Alkinyl, Aryl, Aralkyl und $(C_1-C_4)$-Alkoxy stehen und $R^7$ eine der vorstehend für R unter a) bis f) angegebenen Bedeutungen hat.

Besonders bevorzugt sind Mittel, in weichen in Formel (I) R Wasserstoff, $(C_1-C_4)$-Alkoxy, $(C_2-C_4)$-Alkenyloxy, $(C_2-C_4)$-Alkinyloxy, Benzyloxy, Phenyloxy, NR'R' mit R' = Wasserstoff und/oder $(C_1-C_4)$-Alkyl, Hydrazino oder $-O-CR^3R^3-CO-R^4$ mit $R^3$ = Wasserstoff und $R^4$ = $(C_1-C_4)$-Alkoxy bedeutet, Z für gleiche oder verschiedene Reste aus der Reihe Halogen, insbesondere Fluor und/oder Chlor, $(C_1-C_4)$-Alkyl und $(C_1-C_4)$-Alkoxy steht und n 0, 1 oder 2 ist.

Einige Verbindungen der Formel (I) mit $(Z)_n$ = Wasserstoff und R = $-OCH_3$, $-OC_2H_5$, $-O-CH_2-CH=CH-C_6H_5$, $-OCH_2CH=CHCH_3$, $-OCH_2CH_2CH=CHCH_3$ und Verfahren zu ihrer Herstellung sind aus der Literatur bekannt, ihre Safenerwirkung wurde jedoch nicht erkannt; vgl. z. B. Chem. Pharm. Bull. 28, 3296 (1980); J. Org. Chem. 25, 1160 (1960); J. Org. Chem. 48, 366 (1983); Chem. Lett. 4, 559 (1990); J. Org. Chem. 54, 5277 (1989); Tetrahedron 43, 3983 (1987); Tetrahedron 42, 5267 (1986); Bull. Chem. Soc. Japan 59, 2827 (1986); Bull. Chem. Soc. Japan 58, 2519 (1985); J. Chem. Soc., Chem. Commun. 6, 209 (1976); J. Chem. Soc., Perkin I 1, 437 (1972), Synthesis 1986 und 488-490, Tetrahedron 41 (1985) 727-738. Die vorliegende Erfindung betrifft daher auch neue Verbindungen der Formel I, in welcher X, R, Z und n wie oben definiert sind, und deren Salze, ausgenommen Verbindungen der Formel I, worin X Sauerstoff, R = $-OCH_3$, $-OC_2H_5$, $-O-CH_2-CH = CH-C_6H_5$, $-OCH_2CH = CHCH_3$ oder $-OCH_2CH_2CH = CHCH_3$ und n = 0 bedeuten.

Die neuen Verbindungen der Formel I können analog den in der genannten Literatur beschriebenen Verfahren hergestellt werden. Beispielsweise erhält man Verbindungen der Formel I, indem man ein Styrolderivat der Formel II

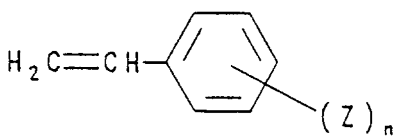

(II)

mit einer Verbindung der Formel III

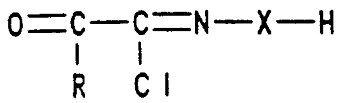

(III)

umsetzt, wobei in den Formeln II und III R, Z, n und X die genannten Bedeutungen haben. Dieses Verfahren ist auch Gegenstand der vorliegenden Erfindung.

Die Umsetzung wird vorzugsweise in einem aprotischen, dipolaren organischen Lösungsmittel wie Ether bei -10°C bis zum Siedepunkt des Reaktionsgemisches und in Gegenwart einer organischen Base wie Triethylamin und Pyridin oder einer anorganischen Base wie Kaliumcarbonat, Natriumcarbonat oder Natriumhydrogencarbonat durchgeführt.

Die Verbindungen der Formel II und III sind bekannt oder lassen sich nach allgemein bekannten Verfahren herstellen (siehe beispielsweise J. Amer. Chem. Soc. 46, 731 (1924); J. Org. Chem. 25, 1160 (1960)).

Die erfindungsgemäßen Verbindungen der Formel I reduzieren oder unterbinden phytotoxische Nebenwirkungen von Herbiziden, die beim Einsatz der Herbizide in Nutzpflanzenkulturen auftreten können, und können deshalb in üblicher Weise als Antidote oder Safener bezeichnet werden. Sie können zusammen mit herbiziden Wirkstoffen oder in beliebiger Reihenfolge ausgebracht werden und sind dann in der Lage, schädliche Nebenwirkungen dieser Herbizide bei Kulturpflanzen zu reduzieren oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Herbizide Wirkstoffe, deren phytotoxische Nebenwirkungen auf Kulturpflanzen mittels Verbindungen der Formel I herabgesetzt werden können, sind z.B. Carbamate, Thiocarbamate, Halogenacetanilide, substituierte Phenoxy-, Naphthoxy- und Phenoxy-phenoxycarbonsäurederivate sowie Heteroaryloxyphenoxyalkancarbonsäurederivate, wie Chinolyloxy-, Chinoxalyloxy-, Pyridyloxy-, Benzoxalyloxy- und Benzthiazolyloxyphenoxyalkancarbonsäureester, Cyclohexandionabkömmlinge, Imidazolinone sowie Sulfonylharnstoffe. Bevorzugt sind dabei Phenoxyphenoxy- und Heteroaryloxy-phenoxycarbonsäureester und -salze, Sulfonylharnstoffe und Imidazolinone.

Geeignete herbizide Wirkstoffe, die mit den erfindungsgemäßen Safenern kombiniert werden können sind

beispielsweise:

A) Wirkstoffe vom Typ der Phenoxyphenoxy- und Heteroaryloxyphenoxycarbonsäure-, ($C_1$-$C_4$)-alkyl-, ($C_2$-$C_4$)-alkenyl- und ($C_3$-$C_4$)-alkinylester wie
A1) Phenoxy-phenoxy- und Benzyloxy-phenoxy-carbonsäure-derivate, z.B.

2-(4-(2,4-Dichlorphenoxy)-phenoxy)-propionsäuremethylester(Diclofop-methyl),
2-(4-(4-Brom-2-chlorphenoxy)-phenoxy)-propionsäuremethylester(s. DE-A-2601548),
2-(4-(4-Brom-2-fluorphenoxy)-phenoxy)-propionsäuremethylester(s. US-A-4808750),
2-(4-(2-Chlor-4-trifluormethylphenoxy)-phenoxy)-propionsäuremethylester(s. DE-A-2433067),
2-(4-(2-Fluor-4-trifluormethylphenoxy)-phenoxy)-propionsäuremethylester(s. US-A-4808750),
2-(4-(2,4-Dichlorbenzyl)-phenoxy)propionsäuremethylester (s. DE-A-2417487),
4-(4-(4-Trifluormethylphenoxy)-phenoxy)-pent-2-en-säureethylester,
2-(4-(4-Trifluormethylphenoxy)-phenoxy)-propionsäuremethylester(s. DE-A-2433067),

A2) "Einkernige" Heteroaryloxy-phenoxy-alkancarbonsäurederivate, z.B.

2-(4-(3,5-Dichlorpyridyl-2-oxy)-phenoxy)-propionsäureethylester(s. EP-A-2925),
2-(4-(3,5-Dichlorpyridyl-2-oxy)-phenoxy)-propionsäurepropargylester (EP-A-3114),
2-(4-(3-Chlor-5-trifluormethyl-2-pyridyloxy)-phenoxy-propionsäure-methylester (s. EP-A-3890),
2-(4-(3-Chlor-5-trifluormethyl-2-pyridyloxy)-phenoxy)-propionsäure-ethylester(s. EP-A-3890),
2-(4-(5-Chlor-3-fluor-2-pyridyloxy)-phenoxy)-propionsäurepropargylester(EP-A-191736),
2-(4-(5-Trifluormethyl-2-pyridyloxy)-phenoxy)-propionsäurebutylester(Fluazifopbutyl),

A3) "Zweikernige" Heteroaryloxy-phenoxy-alkancarbonsäurederivate, z.B.

2-(4-(6-Chlor-2-chinoxalyloxy)-phenoxy)-propionsäuremethylester und - ethylester (Quizalofop-methyl und -ethyl),
2-(4-(6-Fluor-2-chinoxalyloxy)-phenoxy)-propionsäuremethylester (s. J. Pest. Sci. Vol. 10, 61 (1985)),
2-(4-(6-Chlor-2-chinoxalyloxy)-phenoxy)-propionsäure und deren Ester wie der 2-Isopropylidenaminooxyethylester oder der Tetrahydrofurfurylester (Propaquizafop u. Ester),
2-(4-(6-Chlorbenzoxazol-2-yl-oxy)-phenoxy)-propionsäureethylester(Fenoxapropethyl) und
2-(4-(6-Chlorbenzthiazol-2-yloxy)-phenoxypropionsäureethylester (s. DE-A-2640730).

B) Wirkstoffe aus der Sulfonylharnstoff-Reihe, wie z.B. Pyrimidin- oder Triazinylaminocarbonyl-[benzol-, pyridin-, pyrazol-, thiophen- und (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Alkyl, Alkoxyaminocarbonyl, Haloalkoxy, Haloalkyl, Alkylcarbonyl, Alkoxyalkyl, (Alkansulfonyl)alkylamino. Geeignete Sulfonylharnstoffe sind beispielsweise
B1) Phenyl- und Benzylsulfonylharnstoffe und verwandte Verbindungen, z.B.

1-(2-Chlorphenylsulfonyl)-3-(4-methoxy-6-methyl-1,3,5-triazin-2-yl)-harnstoff (Chlorsulfuron),
1-(2-Ethoxycarbonylphenylsulfonyl)-3-(4-chlor-6-methoxypyrimidin-2-yl)-harnstoff (Chlorimuron-ethyl),
1-(2-Methoxyphenylsulfonyl)-3-(4-methoxy-6-methyl-1,3,5-triazin-2-yl)-harnstoff (Metsulfuron-methyl),
1-(2-Chlorethoxy-phenylsulfonyl)-3-(4-methoxy-6-methyl-1,3,5-triazin-2-yl)-harnstoff (Triasulfuron),
1-(2-Methoxycarbonyl-phenylsulfonyl)-3-(4,6-dimethyl-pyrimidin-2-yl)-harnstoff (Sulfometuron-methyl),
1-(2-Methoxycarbonylphenylsulfonyl)-3-(4-methoxy-6-methyl-1,3,5-triazin-2-yl)-3-methylharnstoff (Tribenuron-methyl)
1-(2-Methoxycarbonylbenzylsulfonyl)-3-(4,6-dimethoxy-pyrimidin-2-yl)-harnstoff (Bensulfuron-methyl)
1-(2-Methoxycarbonylphenylsulfonyl)-3-(4,6-bis-(difluormethoxy)-pyrimidin-2-yl)-harnstoff (Pirimisulfuron-methyl),
3-(4-Ethyl-6-methoxy-1,3,5-triazin-2-yl)-1-(2,3-dihydro-1,1-dioxo-2-methylbenzo[b]thiophen-7-sulfonyl)-harnstoff (s. EP-A-79683),
3-(4-Ethoxy-6-ethyl-1,3,5-triazin-2-yl)-1-(2,3-dihydro-1,1-dioxo-2-methylbenzo[b]thiophen-7-sulfonyl)-harnstoff (s. EP-A-79683),

B2) Thienylsulfonylharnstoffe, z.B.

1-(2-Methoxycarbonylthiophen-3-yl)-3-(4-methoxy-6-methyl-1,3,5-triazin-2-yl)-harnstoff (Thifensulfuron-methyl),

B3) Pyrazolylsulfonylharnstoffe, z.B.

1-(4-Ethoxycarbonyl-1-methylpyrazol-5-yl-sulfonyl)-3-(4,6-dimethoxypyrimidin-2-yl)-harnstoff (Pyrazosulfuron-methyl),
Methyl-3-chlor-5-(4,6-dimethoxypyrimidin-2-ylcarbamoylsulfamoyl)-1-methylpyrazol-4-carboxylat (s. EP-A-282613) und
Methyl-5-(4,6-Dimethylpyrimidin-2-yl-carbamoylsulfamoyl)-1-(2-pyridyl)-pyrazol-4-carboxylat (NC 330, siehe Brighton Crop Prot. Conf. Weeds 1991 Vol. 1, s. 45 ff).

B4) Sulfondiamid-Derivate, z.B.

3-(4,6-Dimethoxypyrimidin-2-yl)-1-(N-methyl-N-methylsulfonylaminosulfonyl)-harnstoff (Amidosulfuron) und Strukturanaloge (s. EP-A-0131258 und Z. Pfl. Krankh. Pfl. Schutz, Sonderheft XII, 489-497 (1990)),

B5) Pyridylsulfonylharnstoffe, z.B.

1-(3-N,N-Dimethylaminocarbonylpyridin-2-yl-sulfonyl)-3-(4,6-dimethoxypyrimidin-2-yl)-harnstoff (Nicosulfuron),
1-(3-Ethylsulfonylpyridin-2-yl-sulfonyl)-3-(4,6-dimethoxy-pyrimidin-2-yl)-harnstoff (DPX-E 9636, s. Brighton Crop Prot. Conf. - Weeds - 1989, S. 23 ff.), Pyridylsulfonylharnstoffe, wie sie in den deutschen Patentanmeldungen P 4000503.8 (HOE 90/F 006) und P 4030577.5 (HOE 90/F 293) beschrieben sind, vorzugsweise solche der Formel IV oder deren Salze,

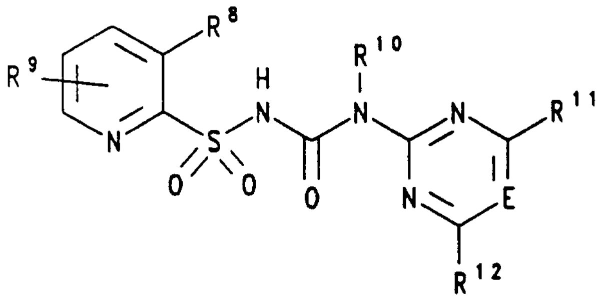

(IV)

worin

- E: CH oder N, vorzugsweise CH,
- $R^8$: Iod oder $NR^{13}R^{14}$,
- $R^9$: Wasserstoff, Halogen, Cyano, $(C_1-C_3)$-Alkyl, $(C_1-C_3)$-Alkoxy, $(C_1-C_3)$-Haloalkyl, $(C_1-C_3)$-Haloalkoxy, $(C_1-C_3)$-Alkylmercapto, $(C_1-C_3)$-Alkoxy-$(C_1-C_3)$-alkyl, $(C_1-C_3)$-Alkoxy-carbonyl, Mono- oder Di-$(C_1-C_3)$-alkyl-amino, $(C_1-C_3)$-Alkyl-sulfinyl oder -sulfonyl, $SO_2-NR^aR^b$ oder $CO-NR^aR^b$, insbesondere Wasserstoff,
- $R^a,R^b$: unabhängig voneinander Wasserstoff, $(C_1-C_3)$-Alkyl, $(C_1-C_3)$-Alkenyl, $(C_1-C_3)$-Alkinyl oder zusammen $-(CH_2)_4-$, $-(CH_2)_5-$ oder $(CH_2)_2-O-(CH_2)_2-$,
- $R^{10}$: Wasserstoff oder $CH_3$,
- $R^{11}$: Halogen, $(C_1-C_2)$-Alkyl, $(C_1-C_2)$-Alkoxy, $(C_1-C_2)$-Haloalkyl, vorzugsweise $CF_3$, $(C_1-C_2)$-Haloalkoxy, vorzugsweise $OCHF_2$ oder $OCH_2CF_3$,
- $R^{12}$: $(C_1-C_2)$-Alkyl, $(C_1-C_2)$-Haloalkoxy, vorzugsweise $OCHF_2$, oder $(C_1-C_2)$-Alkoxy, und
- $R^{13}$: $(C_1-C_4)$-Alkyl und $R^{14}$ $(C_1-C_4)$-Alkylsulfonyl oder $R^{13}$ und $R^{14}$ gemeinsam eine Kette der Formel $-(CH_2)_3SO_2-$ oder $-(CH_2)_4SO_2-$ bedeuten, z.B. 3-(4,6-Dimethoxypyrimidin-2-yl)-1-[(3-(N-methylsulfonyl-N-methylamino)-pyridin-2-yl)-sulfonyl]-harnstoff,

B6) Alkoxyphenoxysulfonylharnstoffe, wie sie in EP-A-0342569 beschrieben sind, vorzugsweise solche der Formel V oder deren Salze,

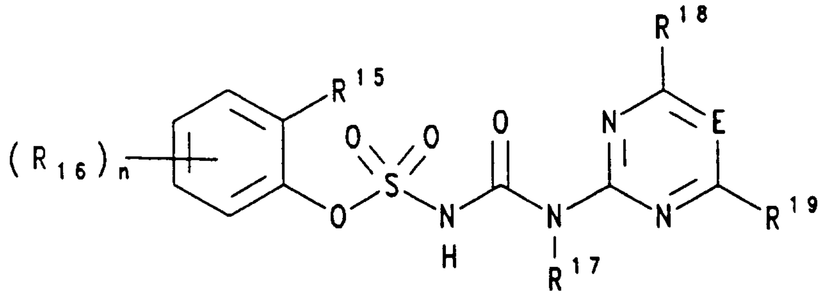

(V)

worin

| | |
|---|---|
| E | CH oder N, vorzugsweise CH, |
| $R^{15}$ | Ethoxy, Propoxy oder Isopropoxy, |
| $R^{16}$ | Wasserstoff, Halogen, Nitro, $CF_3$, CN, ($C_1$-$C_4$)-Alkyl, ($C_1$-$C_4$)-Alkoxy, ($C_1$-$C_4$)-Alkylmercapto oder ($C_1$-$C_3$)-Alkoxy-carbonyl, vorzugsweise in 6-Position am Phenylring, |
| n | 1, 2 oder 3, vorzugsweise 1, |
| $R^{17}$ | Wasserstoff, ($C_1$-$C_4$)-Alkyl oder ($C_3$-$C_4$)-Alkenyl, |
| $R^{18}$,$R^{19}$ | unabhängig voneinander Halogen, ($C_1$-$C_2$)-Alkyl, ($C_1$-$C_2$)-Alkoxy, ($C_1$-$C_2$)-Haloalkyl,$C_1$-$C_2$-Haloalkoxy oder ($C_1$-$C_2$)-Alkoxy-($C_1$-$C_2$)-alkyl, vorzugsweise $OCH_3$ oder $CH_3$, bedeuten, |

z.B. 3-(4,6-Dimethoxypyrimidin-2-yl)-1-(2-ethoxyphenoxy)-sulfonylharnstoff, und andere verwandte Sulfonylharnstoffderivate und Mischungen daraus.

C) Chloracetanilid-Herbizide wie

N-Methoxymethyl-2,6-diethyl-chloracetanilid (Alachlor),
N-(3'-Methoxyprop-2'-yl)-2-methyl-6-ethyl-chloracetanilid (Metolachlor),
N-(3-Methyl-1,2,4-oxadiazol-5-yl-methyl)-chloressigsäure-2,6-dimethylanilid,
N-(2,6-Dimethylphenyl)-N-(1-pyrazolylmethyl)-chloressigsäureamid (Metazachlor),

D) Thiocarbamate wie

S-Ethyl-N,N-dipropylthiocarbamat (EPTC) oder
S-Ethyl-N,N-diisobutylthiocarbamat (Butylate)

E) Cyclohexandion-Derivate wie

Methyl-3-(1-allyloxyimino)butyl)-4-hydroxy-6,6-dimethyl-2-oxocyclohex-3-encarboxylat (Alloxydim)
2-(N-Ethoxybutyrimidoyl)-5-(2-ethylmercaptopropyl)-3-hydroxy-2-cyclohexen-1-on (Sethoxydim),
2-(N-Ethoxybutyrimidoyl)-5-(2-phenylmercaptopropyl)-3-hydroxy-2-cyclohexen-1-on (Cloproxydim),
2-(1-(3-Chlorallyloxy)-iminobutyl)-5-(2-ethylmercapto)-propyl)-3-hydroxy-2-cyclohexen-1-on,
2-(1-(3-Chlorallyloxy)-iminopropyl)-5-(2-ethylmercapto)-propyl)-3-hydroxy-cyclohex-2-en-1-on (Clethodim),
2-(1-Allyloxyiminobutyl)-4-methoxycarbonyl-5,5-dimethyl-3-oxocyclohexenol,
2-(1-(Ethoxyimino)-butyl)-3-hydroxy-5-(thian-3-yl)-cyclohex-2-enon (Cycloxydim), oder
2-(1-Ethoxyiminopropyl)-5-(2,4,6-trimethylphenyl)-3-hydroxy-2-cyclohexen-1-on (Tralkoxydim).

F) Imidazolinone wie 2-Carboxyphenyl- oder 2-Carboxyheteroarylimidazolinone, deren Salze und Ester (z.B. Alkylester), z.B. die Mischung aus

2-(4-Isopropyl-4-methyl-5-oxo-2-imidazolin-2-yl)-5-methylbenzoesäuremethylester und
2-(4-Isopropyl-4-methyl-5-oxo-2-imidazolin-2-yl)-4-methylbenzoesäure (Imazamethabenz), sowie
5-Ethyl-2-(4-isopropyl-4-methyl-5-oxo-2-imidazolin-2-yl)-pyridin-3-carbonsäure, deren Ester und Salze, z.B. das $NH_4$-Salz, (Imazethapyr),

2-[4,5-Dihydro-4-methyl-4-(1-methylethyl)-5-oxo-1H-imidazol-2-yl]-5-methyl-3-pyridincarbonsäure, deren Ester und Salze (Imazethamethapyr) und

2-(4-Isopropyl-4-methyl-5-oxo-2-imidazolin-2-yl)-chinolin-3-carbonsäure, deren Ester und Salze, z.B. das $NH_4$-Salz, (Imazaquin).

Die obengenannten herbiziden Wirkstoffe der Gruppen A bis F sind dem Fachmann bekannt und in der Regel in "The Pesticide Manual", British Crop Protection Council, 9th Edition (1990-91) oder in "Agricultural Chemicals Book II-Herbicides-", by W.T. Thompson, Thompson Publications, Fresno CA, USA 1990 oder in "Farm Chemicals Handbook '90", Meister Publishing Company, Willoughby OH, USA 1990 beschrieben.

Die herbiziden Wirkstoffe und die erwähnten Safener können zusammen (als fertige Formulierung oder im Tankmix-Verfahren) oder in beliebiger Reihenfolge nacheinander ausgebracht werden. Das Gewichtsverhältnis Safener:Herbizid kann innerhalb weiter Grenzen variieren und ist vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle und Sojabohne, vorzugsweise Getreide und Mais.

Ein besonderer Vorteil der erfindungsgemäßen Safener der Formel I ist bei deren Kombination mit Wirkstoffen aus der Gruppe der Sulfonylharnstoffen und/oder Imidazolinone festzustellen. Herbizide der genannten Strukturklassen hemmen primär das Schlüsselenzym Acetolactatsynthase (ALS) in den Pflanzen und sind bezüglich des Wirkungsmechanismus daher zumindest partiell verwandt. Einige Herbizide dieser Strukturklassen können speziell in Getreidekulturen und/oder Mais nicht oder nicht genügend selektiv eingesetzt werden. Durch die Kombination mit den erfindungsgemäßen Safenern sind auch bei diesen Herbiziden in Getreide oder Mais hervorragende Selektivitäten zu erreichen.

Die Safener der Formel I je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und sind in der Regel im Bereich von 0,001 bis 5 kg, vorzugsweise 0,005 bis 0,5 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel I vor, nach oder gleichzeitig mit dem Herbizid auf die Pflanzen, Pflanzenteile, Pflanzensamen oder die Anbaufläche appliziert wird.

Gegenstand der Erfindung sind auch pflanzenschützende Mittel, die einen Wirkstoff der Formel I und übliche Formulierungshilfsmittel enthalten, sowie herbizide Mittel, die einen Wirkstoff der Formel I und einen herbiziden Wirksoff sowie im Bereich des Pflanzenschutzes übliche Formulierungshilfsmittel enthalten.

Die Verbindungen der Formel I und deren Kombinationen mit einem oder mehreren der genannten herbiziden Wirkstoffe können auf verschiedene Art formuliert d.h. in eine für den Pflanzenschutz geeignete Anwendungsform gebracht werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wasserlösliche Pulver (SP), wasserlösliche Konzentrate (SL), konzentrierte Emulsionen (EW) wie Öl-in-Wasser und Wasser-in-Öl-Emulsionen, versprühbare Lösungen oder Emulsionen, Kapselsuspensionen (CS), Dispersionen auf Öl- oder Wasserbasis (SC), Suspoemulsionen, Suspensionskonzentrate, Stäubemittel (DP), ölmischbare Lösungen (OL), Beizmittel, Granulate (GR) in Form von Mikro-, Sprüh-, Aufzugs- und Adsorptionsgranulaten, Granulate für die Boden- bzw. Streuapplikation, wasserlösliche Granulate (SG), wasserdispergierbare Granulate (WG), ULV-Formulierungen, Mikrokapseln und Wachse.

Diese einzelnen Formulierungstypen und Verfahren zu deren Herstellung sind im Prinzip bekannt und werden beispielsweise beschrieben in: Winnacker-Küchler, "Chemische Technologie" Band 7, C. Hauser Verlag München, 4. Aufl. 1986; Wade van Valkenburg, "Pesticide Formulations", Marcel Dekker N.Y., 1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen "Intruduction to Clay Colloid Chemistry", 2nd Ed., J. Wiley & Sons, N.Y., Marsden "Solvents Guide", 2nd Ed., Interscience, N.Y. 1963; McCutcheon's "Detergents and

Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxethylierte Alkylphenole, polyoxethylierte Fettalkohole und Fettamine, Fettalkoholpolyglykolethersulfate, Alkansulfonate oder Alkylarylsulfonate und Dispergiermittel, z.B. ligninsulfonsaures Natrium, 2,2,-dinaphthylmethan-6,6'-disulfonsaures Natrium, dibutylnaphthalinsulfonsaures Natrium oder auch oleoylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyether oder Polyoxethylensorbitanester, z.B. Sorbitanfettsäureester wie Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden z.B. nach üblichen Verfahren wie Sprühtrocknung, Wirbelbett-Granulierung, Teller-Granulierung, Mischung mit Hochgeschwindigkeitsmischern und Extrusionsverfahren ohne festes Inertmaterial hergestellt.

Die agrochemischen Zubereitungen enthalten in der Regel 0,1 bis 99 Gew.-%, insbesondere 0,1 bis 95 Gew.-%, Wirkstoffe der Formel I (Antidot) oder des Antidot/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.%, der Rest zu 100 Gew.-% besteht aus üblichen Formulierungsbestandteilen. Bei emulgierbaren Konzentraten beträgt die Wirkstoffkonzentration etwa 1 bis 80 Gew.-% Wirkstoffe. Staubförmige Formulierungen enthalten etwa 1 bis 20 Gew.-% an Wirkstoffen, versprühbare Lösungen etwa 0,2 bis 20 Gew.-% Wirkstoffe. Bei Granulaten wie wasserdispergierbaren Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Farb- und Trägerstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u.a. variiert die erforderliche Aufwandmenge der "Antidots".

Folgende Beispiele dienen zur Erläuterung der Erfindung, ohne daß diese darauf beschränkt wäre:

A. Formulierungsbeispiele

a) Ein Stäubmittel wird erhalten, indem man 10 Gew.-Teile einer Verbindung der Formel I oder eines Wirkstoffgemischs aus einem Herbizid und eine Verbindung der Formel I und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel I oder eines Wirkstoffgemischs aus einem Herbizid und einem Safener der Formel I, 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel I oder eines Wirkstoffgemischs aus einem Herbizid und einem Safener der Formel I, 6 Gew.-Teilen Alkylphenolpolyglykolether ($^R$Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel I oder eines Wirkstoffgemischs aus einem Herbizid und einem Safener der Formel I, 75 Gew.Teilen Cyclohexanon als Lösemittel und 10 Gew.-Teilen oxethyliertes Nonylphenol als Emulgator.

e) Ein in Wasser dispergierbares Granulat wird erhalten, indem man 75 Gew.-Teile einer Verbindung der Formel I oder eines Wirkstoffgemischs aus einem Herbizid und einem Safener der Formel I,

| | |
|---|---|
| 10 Gew.-Teile | ligninsulfonsaures Calcium, |
| 5 Gew.-Teile | Natriumlaurylsulfat, |
| 3 Gew.-Teile | Polyvinylalkohol und |
| 7 Gew.-Teile | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man 25 Gew.-Teile einer Verbindung der Formel I oder eines Wirkstoffgemischs aus einem Herbizid und einem Safener der Formel I,

| | |
|---|---|
| 5 Gew.-Tole | 2,2'-dinaphthylmethan-6,6'-disulfonsaures Natrium, |
| 2 Gew.-Tole | oleoylmethyltaurinsaures Natrium, |
| 1 Gew.-Tole | Polyvinylalkohol, |
| 17 Gew.-Tole | Calciumcarbonat und |
| 50 Gew.-Tole | Wasser auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet. |

B. Chemische Beispiele

5-(2-Methoxyphenyl)-2-isoxazolin-3-carbonsäureethylester (Beispiel 125, s. Tabelle 1)

4,2 g 2,4-Difluorstyrol und 4,55 g 2-Chlor-2-hydroximino-essigsäureethylester werden in 350 ml Ether vorgelegt und auf 0°C gekühlt. Dazu tropft man bei 0°C 3,03 g Triethylamin. Nach 3-stündigem Rühren bei Raumtemperatur werden 50 ml Wasser zugegeben und das Gemisch mit Ether extrahiert. Nach Trocknen über $MgSO_4$ wird der Ether abdestilliert und der Rückstand über eine Kieselgelsäule (Laufmittel: n-Heptan : Essigester = 8 : 2) gereinigt. So wurden 6,58 g (86 % d. Th.) Produkt mit dem Brechungsindex $[n]_D^{20}$ = 1,5019 erhalten.

Die Derivate der folgenden Tabelle 1 werden auf analogem Weg erhalten.

Tabelle 1:

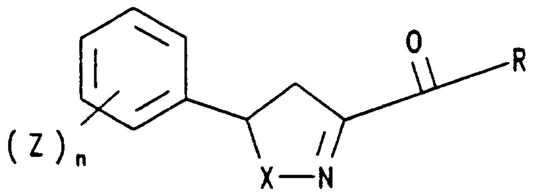

| Beisp. Nr. | $(Z)_n$ | R | X | Schmp./$[n]_D^{20}$ |
|---|---|---|---|---|
| 1 | n=0 | OH | O | |
| 2 | n=0 | $OCH_3$ | O | 40°C |
| 3 | n=0 | $OC_2H_5$ | O | 1,5331 |
| 4 | n=0 | n-$OC_3H_7$ | O | |
| 5 | n=0 | i-$OC_3H_7$ | O | |
| 6 | n=0 | n-$OC_4H_9$ | O | |
| 7 | n=0 | $OCH_2CO_2C_2H_5$ | O | |
| 8 | n=0 | $OC_6H_5$ | O | |
| 9 | n=0 | $OCH_2C_6H_5$ | O | |
| 10 | n=0 | $OCH_2CH=CH_2$ | O | |
| 11 | n=0 | $OCH_2C\equiv CH$ | O | |
| 12 | n=0 | $OCH_2Si(CH_3)_3$ | O | |
| 13 | n=0 | $O^-K^+$ | O | |
| 14 | 2-Cl | OH | O | |
| 15 | 2-Cl | $OCH_3$ | O | |
| 16 | 2-Cl | $OC_2H_5$ | O | |
| 17 | 2-Cl | n-$OC_3H_7$ | O | |
| 18 | 2-Cl | i-$OC_3H_7$ | O | |
| 19 | 2-Cl | n-$OC_4H_9$ | O | |
| 20 | 2-Cl | $OCH_2CO_2C_2H_5$ | O | |
| 21 | 2-Cl | $OC_6H_5$ | O | |

Fortsetzung von Tabelle 1

| Beisp. Nr. | $(Z)_n$ | R | X | Schmp./$[n]_D^{20}$ |
|---|---|---|---|---|
| 22 | 2-Cl | $OCH_2C_6H_5$ | O | |
| 23 | 2-Cl | $OCH_2CH=CH_2$ | O | |
| 24 | 2-Cl | $OCH_2C\equiv CH$ | O | |
| 25 | 2-Cl | $OCH_2Si(CH_3)_3$ | O | |
| 26 | 2-Cl | $O^-K^+$ | O | |
| 27 | 4-Cl | OH | O | |
| 28 | 4-Cl | $OCH_3$ | O | 78°C |
| 29 | 4-Cl | $OC_2H_5$ | O | 58°C |
| 30 | 4-Cl | n-$OC_3H_7$ | O | |
| 31 | 4-Cl | i-$OC_3H_7$ | O | |
| 32 | 4-Cl | n-$OC_4H_9$ | O | |
| 33 | 4-Cl | $OCH_2CO_2C_2H_5$ | O | |
| 34 | 4-Cl | $OC_6H_5$ | O | |
| 35 | 4-Cl | $OCH_2C_6H_5$ | O | |
| 36 | 4-Cl | $OCH_2CH=CH_2$ | O | |
| 37 | 4-Cl | $OCH_2C\equiv CH$ | O | |
| 38 | 4-Cl | $OCH_2Si(CH_3)_3$ | O | |
| 39 | 2,4-$Cl_2$ | OH | O | |
| 40 | 2,4-$Cl_2$ | $OCH_3$ | O | 83-84°C |
| 41 | 2,4-$Cl_2$ | $OC_2H_5$ | O | 75-76°C |
| 42 | 2,4-$Cl_2$ | n-$OC_3H_7$ | O | |
| 43 | 2,4-$Cl_2$ | i-$OC_3H_7$ | O | |
| 44 | 2,4-$Cl_2$ | n-$OC_4H_9$ | O | |
| 45 | 2,4-$Cl_2$ | $OCH_2CO_2C_2H_5$ | O | |
| 46 | 2,4-$Cl_2$ | $OC_6H_5$ | O | |
| 47 | 2,4-$Cl_2$ | $OCH_2C_6H_5$ | O | |
| 48 | 2,4-$Cl_2$ | $OCH_2CH=CH_2$ | O | |
| 49 | 2,4-$Cl_2$ | $OCH_2C\equiv CH$ | O | |
| 50 | 2,4-$Cl_2$ | $OCH_2Si(CH_3)_3$ | O | |

Fortsetzung von Tabelle 1

| Beisp. Nr. | $(Z)_n$ | R | X | Schmp./$[n]_D^{20}$ |
|---|---|---|---|---|
| 51 | 2,4-$Cl_2$ | $OCH_2CO_2CH_3$ | O | |
| 52 | 2,6-$Cl_2$ | OH | O | |
| 53 | 2,6-$Cl_2$ | $OCH_3$ | O | 116-117°C |
| 54 | 2,6-$Cl_2$ | $OC_2H_5$ | O | 105-106°C |
| 55 | 2,6-$Cl_2$ | n-$OC_3H_7$ | O | |
| 56 | 2,6-$Cl_2$ | i-$OC_3H_7$ | O | |
| 57 | 2,6-$Cl_2$ | n-$OC_4H_9$ | O | |
| 58 | 2,6-$Cl_2$ | $OCH_2CO_2C_2H_5$ | O | |
| 59 | 2,6-$Cl_2$ | $OC_6H_5$ | O | |
| 60 | 2,6-$Cl_2$ | $OCH_2C_6H_5$ | O | |
| 61 | 2,6-$Cl_2$ | $OCH_2CH=CH_2$ | O | |
| 62 | 2,6-$Cl_2$ | $OCH_2C\equiv CH$ | O | |
| 63 | 2,6-$Cl_2$ | $OCH_2Si(CH_3)_3$ | O | |
| 64 | 4-$OCH_3$ | OH | O | |
| 65 | 4-$OCH_3$ | $OCH_3$ | O | 71°C |
| 66 | 4-$OCH_3$ | $OC_2H_5$ | O | 1,5402 |
| 67 | 4-$OCH_3$ | n-$OC_3H_7$ | O | |
| 68 | 4-$OCH_3$ | i-$OC_3H_7$ | O | |
| 69 | 4-$OCH_3$ | n-$OC_4H_9$ | O | |
| 70 | 4-$OCH_3$ | $OCH_2CO_2C_2H_5$ | O | |
| 71 | 4-$OCH_3$ | $OC_6H_5$ | O | |
| 72 | 4-$OCH_3$ | $OCH_2C_6H_5$ | O | |
| 73 | 4-$OCH_3$ | $OCH_2CH=CH_2$ | O | |
| 74 | 4-$OCH_3$ | $OCH_2C\equiv CH$ | O | |
| 75 | 4-$OCH_3$ | $OCH_2Si(CH_3)_3$ | O | |
| 76 | 2-$OCH_3$ | OH | O | |
| 77 | 2-$OCH_3$ | $OCH_3$ | O | |
| 78 | 2-$OCH_3$ | $OC_2H_5$ | O | |
| 79 | 2-$OCH_3$ | n-$OC_3H_7$ | O | |

Fortsetzung von Tabelle 1

| Beisp. Nr. | $(Z)_n$ | R | X | Schmp./$[n]_D^{20}$ |
|---|---|---|---|---|
| 80 | 2-$OCH_3$ | i-$OC_3H_7$ | O | |
| 81 | 2-$OCH_3$ | n-$OC_4H_9$ | O | |
| 82 | 2-$OCH_3$ | $OCH_2CO_2C_2H_5$ | O | |
| 83 | 2-$OCH_3$ | $OC_6H_5$ | O | |
| 84 | 2-$OCH_3$ | $OCH_2C_6H_5$ | O | |
| 85 | 2-$OCH_3$ | $OCH_2CH=CH_2$ | O | |
| 86 | 2-$OCH_3$ | $OCH_2C \equiv CH$ | O | |
| 87 | 2-$OCH_3$ | $OCH_2Si(CH_3)_3$ | O | |
| 88 | 2-$CH_3$ | OH | O | |
| 89 | 2-$CH_3$ | $OCH_3$ | O | |
| 90 | 2-$CH_3$ | $OC_2H_5$ | O | |
| 91 | 2-$CH_3$ | n-$OC_3H_7$ | O | |
| 92 | 2-$CH_3$ | i-$OC_3H_7$ | O | |
| 93 | 2-$CH_3$ | n-$OC_4H_9$ | O | |
| 94 | 2-$CH_3$ | $OCH_2CO_2C_2H_5$ | O | |
| 95 | 2-$CH_3$ | $OC_6H_5$ | O | |
| 96 | 2-$CH_3$ | $OCH_2C_6H_5$ | O | |
| 97 | 2-$CH_3$ | $OCH_2CH=CH_2$ | O | |
| 98 | 2-$CH_3$ | $OCH_2C \equiv CH$ | O | |
| 99 | 2-$CH_3$ | $OCH_2Si(CH_3)_3$ | O | |
| 100 | 2-$CH_3$ | $OCH_3$ | S | |
| 101 | 2-$CH_3$ | $OC_2H_5$ | S | |
| 102 | 4-Cl | $OCH_3$ | S | |
| 103 | 4-Cl | $OC_2H_5$ | S | |
| 104 | 2,4-$Cl_2$ | $OCH_3$ | S | |
| 105 | 2,4-$Cl_2$ | $OC_2H_5$ | S | |
| 106 | 2,6-$Cl_2$ | $OCH_3$ | S | |
| 107 | 2,6-$Cl_2$ | $OC_2H_5$ | S | |
| 108 | 4-$OCH_3$ | $OCH_3$ | S | |

Fortsetzung von Tabelle 1

| Beisp. Nr. | $(Z)_n$ | R | X | Schmp./$[n]_D^{20}$ |
|---|---|---|---|---|
| 109 | 4-$OCH_3$ | $OC_2H_5$ | S | |
| 110 | 2-$OCH_3$ | $OCH_3$ | S | |
| 111 | 2-$OCH_3$ | $OC_2H_5$ | S | |
| 112 | 2-$CH_3$ | $OCH_3$ | S | |
| 113 | 2-$CH_3$ | $OC_2H_5$ | S | |
| 114 | 2-Cl | $N(CH_3)_2$ | O | |
| 115 | 2-Cl | $NHNH_2$ | O | |
| 116 | 2-Cl | $NH_2$ | O | |
| 117 | 2,4-$Cl_2$ | $N(CH_3)_2$ | O | |
| 118 | 2,4-$Cl_2$ | $NHNH_2$ | O | |
| 119 | 2,4-$Cl_2$ | $NH_2$ | O | |
| 120 | 4-Cl | $N(CH_3)_2$ | O | |
| 121 | 4-Cl | $NHNH_2$ | O | |
| 122 | 4-Cl | $NH_2$ | O | |
| 123 | 2,4-$F_2$ | OH | O | 142°C (Zers.) |
| 124 | 2,4-$F_2$ | $OCH_3$ | O | 1,5101 |
| 125 | 2,4-$F_2$ | $OC_2H_5$ | O | 1,5019 |
| 126 | 2,4-$F_2$ | n-$OC_3H_7$ | O | |
| 127 | 2,4-$F_2$ | i-$OC_3H_7$ | O | |
| 128 | 2,4-$F_2$ | n-$OC_4H_9$ | O | |
| 129 | 2,4-$F_2$ | $OCH_2CO_2C_2H_5$ | O | |
| 130 | 2,4-$F_2$ | $OC_6H_5$ | O | |
| 131 | 2,4-$F_2$ | $OCH_2C_6H_5$ | O | |
| 132 | 2,4-$F_2$ | $OCH_2CH{=}CH_2$ | O | |
| 133 | 2,4-$F_2$ | $OCH_2C{\equiv}CH$ | O | |
| 134 | 2,4-$F_2$ | $-O^-K^+$ | O | |
| 135 | 2,4-$F_2$ | $-O^-Na^+$ | O | |
| 136 | 3-Cl | OH | O | |
| 137 | 3-Cl | $OCH_3$ | O | 53°C |

Fortsetzung von Tabelle 1

| Beisp. Nr. | $(Z)_n$ | R | X | Schmp./$[n]_D^{20}$ |
|---|---|---|---|---|
| 138 | 3-Cl | $OC_2H_5$ | O | 1,5305 |
| 139 | 3-Cl | n-$OC_3H_7$ | O | |
| 140 | 3-Cl | i-$OC_3H_7$ | O | |
| 141 | 3-Cl | n-$OC_4H_9$ | O | |
| 142 | 3-Cl | $OCH_2CO_2C_2H_5$ | O | |
| 143 | 3-Cl | $OC_6H_5$ | O | |
| 144 | 3-Cl | $OCH_2C_6H_5$ | O | |
| 145 | 3-Cl | $OCH_2CH=CH_2$ | O | |
| 146 | 3-Cl | $OCH_2C\equiv CH$ | O | |
| 147 | 3-Cl | $-O^-K^+$ | O | |
| 148 | 3-Cl | $-O^-Na^+$ | O | |
| 149 | 4-$CH_3$ | OH | O | |
| 150 | 4-$CH_3$ | $OCH_3$ | O | 1,5370 |
| 151 | 4-$CH_3$ | $OC_2H_5$ | O | 52°C |
| 152 | 4-$CH_3$ | n-$OC_3H_7$ | O | |
| 153 | 4-$CH_3$ | i-$OC_3H_7$ | O | |
| 154 | 4-$CH_3$ | n-$OC_4H_9$ | O | |
| 155 | 4-$CH_3$ | $OCH_2CO_2C_2H_5$ | O | |
| 156 | 4-$CH_3$ | $OC_6H_5$ | O | |
| 157 | 4-$CH_3$ | $OCH_2C_6H_5$ | O | |
| 158 | 4-$CH_3$ | $OCH_2CH=CH_2$ | O | |
| 159 | 4-$CH_3$ | $OCH_2C\equiv CH$ | O | |
| 160 | 4-$CH_3$ | $-O^-K^+$ | O | |
| 161 | 4-$CH_3$ | $-O^-Na^+$ | O | |
| 162 | 2,6-$(OCH_3)_2$ | OH | O | |
| 163 | 2,6-$(OCH_3)_2$ | $OCH_3$ | O | 1,5529 |
| 164 | 2,6-$(OCH_3)_2$ | $OC_2H_5$ | O | 1,5438 |
| 165 | 2,6-$(OCH_3)_2$ | n-$OC_3H_7$ | O | |
| 166 | 2,6-$(OCH_3)_2$ | i-$OC_3H_7$ | O | |

Fortsetzung von Tabelle 1

| Beisp. Nr. | $(Z)_n$ | R | X | Schmp./$[n]_D^{20}$ |
|---|---|---|---|---|
| 167 | 2,6-$(OCH_3)_2$ | n-$OC_4H_9$ | O | |
| 168 | 2,6-$(OCH_3)_2$ | $OCH_2CO_2C_2H_5$ | O | |
| 169 | 2,6-$(OCH_3)_2$ | $OC_6H_5$ | O | |
| 170 | 2,6-$(OCH_3)_2$ | $OCH_2C_6H_5$ | O | |
| 171 | 2,6-$(OCH_3)_2$ | $OCH_2CH=CH_2$ | O | |
| 172 | 2,6-$(OCH_3)_2$ | $OCH_2C\equiv CH$ | O | |
| 173 | 2,6-$(OCH_3)_2$ | $-O^-K^+$ | O | |
| 174 | 2,6-$(OCH_3)_2$ | $-O^-Na^+$ | O | |

C. Biologische Beispiele

Beispiel 1

Weizen und Gerste wurden im Gewächshaus in Plastiktöpfen bis zum 3-4 Blattstadium herangezogen und dann nacheinander mit den erfindungsgemäßen Verbindungen und den getesteten herbiziden Wirkstoffe im Nachauflaufverfahren behandelt. Die herbiziden Wirkstoffe und die Verbindungen der Formel I wurden dabei in Form wäßriger Suspensionen bzw. Emulsionen mit einer Wasseraufwandmenge von umgerechnet 300 bis 600 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse aus Tabelle 2 veranschaulichen, daß die erfindungsgemäßen Verbindungen starke Herbizidschäden an Kulturpflanzen effektiv reduzieren können.

Selbst bei starken Überdosierungen des Herbizids werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

Tabelle 2

| Safenerwirkung der erfindungsgemäßen Verbindungen | | | | |
|---|---|---|---|---|
| Bsp. | Nr. | kg a.i./ha | TRAE | HOVU |
| H | | 2,0 | 80 | -- |
| | | 0,2 | -- | 85 |
| H+ | 124 | 2,0 + 1,25 | 20 | -- |
| H+ | 124 | 0,2 + 1,25 | -- | 30 |
| H+ | 137 | 2,0 + 1,25 | 25 | -- |

Tabelle 2 (fortgesetzt)

| Safenerwirkung der erfindungsgemäßen Verbindungen | | | | |
|---|---|---|---|---|
| Bsp. | Nr. | kg a.i./ha | TRAE | HOVU |
| H+ | 137 | 0,2 + 1,25 | -- | 35 |

Abkürzungen:
TRAE = Triticum aestivum
HOVU = Hordeum vulgare
a.i. = Aktivsubstanz
Nr. = Verbindung aus Tabelle 1 mit derselben Nummer
H = 2-(4-(6-Chlorbenzoxazol-2-yloxy)-phenoxypropionsäureethylester (Fenoxaprop-ethyl)
In der Spalte unter TRAE bzw. HOVU sind die Schadwirkungen (Herbizidwirkungen) in Prozentwerten angegeben (100 = Pflanze stirbt ab, O = keine Schäden).

Vergleichbare Ergebnisse in der kulturpflanzenschützenden Wirkung werden beispielsweise mit den Verbindungen der Beispiele 2, 3, 28, 29, 41, 125 und 138 aus Tabelle 1 in Getreide bei einer Aufwandmenge zwischen 0,01 und 1,5 kg/ha Aktivsubstanz Safener im Nachauflaufverfahren erhalten.

Beispiel 2

Maispflanzen, Unkräuter und Ungräser werden im Freiland oder im Gewächshaus in Plastiktöpfen bis zum 4- bis 5-Blattstadium herangezogen und nacheinander mit Herbiziden und erfindungsgemäßen Verbindungen der Formel I im Nachauflaufverfahren behandelt. Die Wirkstoffe werden dabei in Form wäßriger Suspensionen bzw. Emulsionen mit einer Wasseraufwandmenge von umgerechnet 300 bis 600 l/ha ausgebracht. 4 Wochen nach der Behandlung werden die Pflanzen visuell auf jede Art von Schädigung durch die ausgebrachten Herbizide bonitiert, wobei insbesondere das Ausmaß der anhaltenden Wachstumshemmung berücksichtigt wird. Die Bewertung erfolgt in Prozentwerten im Vergleich zu unbehandelten Kontrollen.

Die Ergebnisse zeigen, daß die erfindungsgemäß eingesetzten Verbindungen der Formel I starke Herbizidschäden an den Maispflanzen effektiv reduzieren können. Selbst bei starken Überdosierungen der Herbizide werden bei den Kulturpflanzen auftretende schwere Schädigungen deutlich reduziert und geringere Schäden völlig aufgehoben. Beispielsweise werden mit 0,01 bis 1,5 kg/ha Aktivsubstanz der Verbindungen der Beispiele 2, 3, 28, 29, 41, 124, 125, 137 oder 138 aus Tabelle 1 gute Safenerwirkungen an Mais bei kombinierter Anwendung mit dem Herbizid 1-[3-(N,N-Dimethylaminocarbonyl)-pyridin-2-ylsulfonyl]-3-(4,6-dimethoxypyrimidin-2-yl)-harnstoff (Nicosulfuron),

3-(4,6-Dimethoxypyrimidin-2-yl)-1-[3-(N-methyl-N-methylsulfonyl-amino)-2-pyridyl-sulfonyl]-harnstoff,
1-(3-Ethylsulfonylpyridin-2-yl-sulfonyl)-3-(4,6-dimethoxy-pyrimidin-2-yl)-harnstoff (DPX-E 9636),
5-Ethyl-2-(4-isopropyl-4-methyl-5-oxo-2-imidazolin-2-yl)-pyridin-3-carbonsäureammoniumsalz (Imazethapyr-ammonium) oder
1-(2-Methoxycarbonylphenylsulfonyl)-3-(4,6-bis-(difluormethoxy)-pyrimidin-2-yl)-harnstoff (Primisulfuron-methyl) erreicht.

Mischungen aus Herbiziden und Verbindungen der Formel I eignen sich deshalb in ausgezeichneter Weise zur selektiven Unkrautbekämpfung in Mais.

**Patentansprüche**

**Patentansprüche für folgende Vertragsstaaten : AT, BE, CH, LI, DE, DK, FR, GB, IT, NL, PT, SE**

**1.** Kulturpflanzenschützendes Mittel, welches mindenstens eine Verbindung der Formel I oder deren Salz,

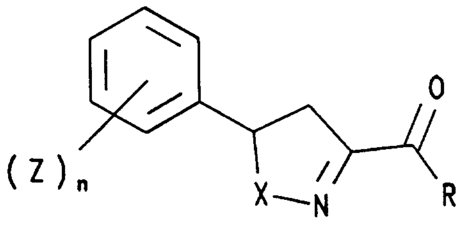

(I)

in welcher

X ein Sauerstoff- oder Schwefelatom bedeutet,

R

a) Hydroxy, Mercapto, $(C_1\text{-}C_5)$Alkoxy, $(C_2\text{-}C_5)$Alkenyloxy, $(C_2\text{-}C_5)$Alkinyloxy, $(C_1\text{-}C_5)$Alkylmercapto, $(C_2\text{-}C_5)$Alkenylmercapto, $(C_2\text{-}C_5)$Alkinylmercapto, $(C_3\text{-}C_7)$Cycloalkyloxy oder $(C_3\text{-}C_7)$Cycloalkylmercapto bedeutet, wobei die 8 letztgenannten Gruppen unsubstituiert oder mit einem oder mehreren gleichen oder verschiedenen Resten aus der Reihe $(C_6\text{-}C_{12})$Aryl, $(C_1\text{-}C_5)$Alkoxy, $(C_2\text{-}C_5)$Alkenyloxy, $(C_2\text{-}C_5)$Alkinyloxy, $(C_6\text{-}C_{12})$Aralkyloxy, $(C_6\text{-}C_{12})$Aryloxy, $(C_3\text{-}C_7)$Cycloalkyloxy, $(C_1\text{-}C_5)$Alkylmercapto, Mono- oder Di-$(C_1\text{-}C_5)$alkylamino, Cyano, Halogen und Nitro substituiert sind,
b) $(C_6\text{-}C_{12})$Aralkyloxy, $(C_6\text{-}C_{12})$Aryloxy, $(C_6\text{-}C_{12})$Aralkylmercapto oder $(C_6\text{-}C_{12})$Arylmercapto, welche jeweils unsubstituiert oder mit einem oder mehreren gleichen oder verschiedenen Resten aus der Reihe $(C_1\text{-}C_5)$Alkyl, $(C_2\text{-}C_5)$Alkenyl, $(C_2\text{-}C_5)$Alkinyl, Halogen, Cyano, Nitro, $(C_1\text{-}C_5)$Alkoxy, $(C_2\text{-}C_5)$Alkenyloxy, $(C_2\text{-}C_5)$Alkinyloxy, $(C_1\text{-}C_5)$Alkylmercapto, Mono- oder Di-$(C_1\text{-}C_5)$alkylamino, $(C_6\text{-}C_{12})$Aryloxy und $(C_6\text{-}C_{12})$Aroyloxy substituiert sind, oder Tri-$(C_1\text{-}C_5)$alkylsilyl-$(C_1\text{-}C_5)$alkoxy, $(C_6\text{-}C_{12})$Aryldi-$(C_1\text{-}C_5)$alkylsilyloxy, $(C_6\text{-}C_{12})$Aryl$(C_1\text{-}C_5)$alkyldi-$(C_1\text{-}C_5)$alkylsilyloxy, Di-$(C_6\text{-}C_{12})$aryl$(C_1\text{-}C_5)$alkylsilyloxy oder Di-[$(C_6\text{-}C_{12})$aryl$(C_1\text{-}C_5)$alkyl]-$(C_1\text{-}C_5)$alkylsilyloxy bedeutet,
c) einen Rest der Formel NR'R', worin die R' für gleiche oder verschiedene Reste aus der Gruppe Wasserstoff, $(C_1\text{-}C_5)$Alkyl, $(C_2\text{-}C_5)$Alkenyl, $(C_2\text{-}C_5)$Alkinyl und $(C_3\text{-}C_7)$Cycloalkyl steht, oder Pyridino, Morpholino, Di-$(C_1\text{-}C_5)$alkylmorpholino, Hydrazino oder einen Rest der Formel

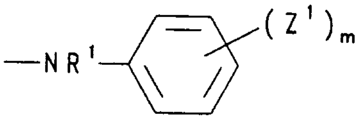

bedeutet,
worin $R^1$ Wasserstoff, $(C_1\text{-}C_5)$Alkyl, $(C_2\text{-}C_5)$Alkenyl oder $(C_2\text{-}C_5)$Alkinyl, die Reste $Z^1$ unabhängig voneinander Halogen, Nitro, $(C_1\text{-}C_5)$Alkyl, $(C_2\text{-}C_5)$Alkenyl, $(C_1\text{-}C_5)$Alkoxy oder $(C_6\text{-}C_{12})$Aryloxy und m eine ganze Zahl von 0 bis 5 sind,
d) einen Rest der Formel

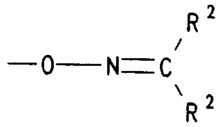

bedeutet, worin die Reste $R^2$ unabhängig voneinander $(C_1\text{-}C_5)$Alkyl oder gemeinsam mit dem sie verknüpfenden C-Atom $(C_3\text{-}C_7)$Cycloalkyliden bedeuten,
e) einen Rest der Formel $\text{-O-CR}^3\text{R}^3\text{-CO-R}^4$ bedeutet, worin $R^3$ für gleiche oder verschiedene Reste aus der Reihe Wasserstoff, $(C_1\text{-}C_5)$Alkyl, $(C_2\text{-}C_5)$Alkenyl, $(C_2\text{-}C_5)$Alkinyl, $(C_6\text{-}C_{12})$Aryl, $(C_6\text{-}C_{12})$Aryl $(C_1\text{-}C_5)$alkyl, $(C_1\text{-}C_5)$Alkoxy, $(C_2\text{-}C_5)$Alkenyloxy, $(C_2\text{-}C_5)$Alkinyloxy und $(C_6\text{-}C_{12})$Aryloxy steht und $R^4$ für Wasserstoff, $(C_1\text{-}C_5)$Alkyl, $(C_2\text{-}C_5)$Alkenyl, $(C_2\text{-}C_5)$Alkinyl, $(C_6\text{-}C_{12})$Aryl oder $(C_6\text{-}C_{12})$Aryl-$(C_1\text{-}C_5)$alkyl steht,

f) einen Rest der Formel

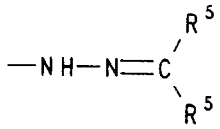

bedeutet, worin $R^5$ für gleiche oder verschiedene Reste aus der Reihe Wasserstoff, $(C_1-C_5)$Alkyl und $(C_6-C_{12})$Aryl steht oder die beiden Reste $R^5$ gemeinsam mit dem sie Verknüpfenden C-Atom $(C_3-C_7)$Cycloalkyliden bedeuten, oder

g) einen Rest der Formel $-O-CR^6R^6-CO-R^7$ bedeutet, worin $R^6$ für gleiche oder verschiedene Reste aus der Reihe Wasserstoff, $(C_1-C_5)$Alkyl, $(C_2-C_5)$Alkenyl, $(C_2-C_5)$Alkinyl, $(C_{6-12})$Aryl, $(C_6-C_{12})$Aryl-$(C_1-C_5)$alkyl, $(C_1-C_5)$Alkoxy, $(C_2-C_5)$Alkenyloxy, $(C_2-C_5)$Alkinyloxy und $(C_6-C_{12})$Aryloxy steht und $R^7$ eine der vorstehend für R unter a) bis f) angegebenen Bedeutungen hat,

Z Halogen, Nitro, Cyano, $(C_1-C_4)$-Alkyl, $(C_1-C_4)$-Alkoxy, $(C_1-C_4)$-Alkylmercapto, wobei die Alkyl-, Alkoxy- und Alkylmercaptogruppen unabhängig voneinander jeweils unsubstituiert oder mit einem oder mehreren Halogenatomen substituiert sind, $(C_3-C_6)$-Cycloalkyl, das unsubstituiert oder mit vorzugsweise bis zu drei $(C_1-C_4)$-Alkyl substituiert ist, Amino, Hydroxymethyl, $(C_1-C_4)$-Alkylamino, Di-$(C_1-C_4)$-alkylamino, $(C_1-C_4)$-Alkoxymethyl, wobei die Alkyl- und Alkoxygruppen in den drei letztgenannten Resten unabhängig voneinander unsubstituiert oder mit vorzugsweise bis zu drei $(C_1-C_4)$-Alkyl substituiert sind, $(C_6-C_{12})$Aryl oder $(C_6-C_{12})$Aryloxy, wobei Aryl und Aryloxy unabhängig voneinander jeweils unsubstituiert oder ein- oder mehrfach gleichen oder verschiedenen Resten aus der Reihe Halogen und Trifiuormethyl substituiert sind, bedeutet und

n eine ganze Zahl von 0 bis 5 ist,

und 0,1 bis 25 Gew.-%, bezogen auf das Mittel, Tensid enthält.

**2.** Mittel gemäß Anspruch 1, in welchem in Formel I

R

a) Hydroxy, Mercapto, $(C_1-C_4)$-Alkoxy, $(C_2-C_4)$-Alkenyloxy, $(C_2-C_4)$-Alkinyloxy, $(C_1-C_4)$-Alkylmercapto, $(C_2-C_4)$-Alkenylmercapto, $(C_2-C_4)$-Alkinylmercapto oder $(C_3-C_8)$-Cycloalkylmercapto bedeutet, wobei die 8 letztgenannten Gruppen unsubstituiert oder mit einem oder mehreren gleichen oder verschiedenen Resten aus der Reihe Aryl, $(C_1-C_4)$-Alkoxy, $(C_2-C_4)$-Alkenyloxy, $(C_2-C_4)$-Alkinyloxy, Aralkyloxy, Aryloxy, $(C_3-C_8)$-Cycloalkyloxy, $(C_1-C_4)$-Alkylmercapto, Mono- oder Di-$(C_1-C_4)$-Alkylamino, Cyano, Halogen und Nitro substituiert sind,

b) Aryloxy, Arylmercapto, Aralkyloxy oder Aralkylmercapto, welche jeweils unsubstituiert oder mit einem oder mehreren gleichen oder verschiedenen Resten aus der Reihe $(C_1-C_4)$-Alkyl, $(C_2-C_4)$-Alkenyl, $(C_2-C_4)$-Alkinyl, Halogen, Cyano, Nitro, $(C_1-C_4)$-Alkoxy, $(C_2-C_4)$-Alkenyloxy, $(C_2-C_4)$-Alkinyloxy, $(C_1-C_4)$-Alkylmercapto, Mono- oder Di-$(C_1-C_4)$-Alkylamino, Aryloxy und Aralkyloxy substituiert sind, oder Tri-$(C_1-C_4)$-alkylsilylalkoxy bedeutet,

c) einen Rest der Formel -NR'R', worin R' Wasserstoff und/oder $(C_1-C_4)$-Alkyl bedeutet, Pyridino, Morpholino, Dimethylmorpholino, Hydrazino oder einen Rest der Formel

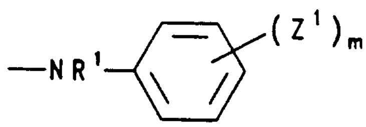

bedeutet, worin $R^1$ Wasserstoff oder $(C_1-C_4)$-Alkyl, die Reste $Z^1$ unabhängig voneinander Halogen, Nitro, $(C_1-C_4)$-Alkyl, $(C_1-C_4)$-Alkoxy oder Aryloxy und m 0 bis 3 sind,

d) einen Rest der Formel $-O-N=CR^2R^2$ bedeutet, worin $R^2$ für $(C_1-C_4)$-Alkyl steht oder die Reste $R^2$ gemeinsam mit dem sie verknüpfenden C-Atom für Cyclohexyliden oder Cyclopentyliden stehen,

e) einen Rest der Formel $-O-CR^3R^3-CO-R^4$, worin $R^3$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Aryl, Aralkyl oder ($C_1$-$C_4$)-Alkoxy steht und $R^4$ für ($C_1$-$C_4$)-Alkyl, ($C_2$-$C_4$)-Alkenyl, ($C_2$-$C_4$)-Alkinyl, Aryl oder Aralkyl steht,

f) einen Rest der Formel $-NH-N=CR^5R^5$, worin $R^5$ für gleiche oder verschiedene Reste aus der Reihe Wasserstoff, ($C_1$-$C_4$)-Alkyl oder Aryl oder die beiden Reste $R^5$ gemeinsam mit dem sie verknüpfenden C-Atom Cyclohexyliden oder Cyclopentyliden bedeuten, oder

g) einen Rest der Formel $-O-CR^6R^6-CO-R^7$ bedeutet, worin $R^6$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Aryl, Aralkyl und ($C_1$-$C_4$)-Alkoxy stehen und $R^7$ eine der vorstehend für R unter a) bis f) angegebenen Bedeutungen hat.

**3.** Mittel gemäß Anspruch 1 oder 2, in welchem in Formel I

R

a) Hydroxy, Mercapto, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, ($C_2$-$C_4$)-Alkenylmercapto, ($C_2$-$C_4$)-Alkinylmercapto oder ($C_3$-$C_8$)-Cycloalkylmercapto bedeutet, wobei die 8 letztgenannten Gruppen unsubstituiert oder mit bis zu drei gleichen oder verschiedenen Resten aus der Reihe Phenyl, ($C_1$-$C_4$)-Alkoxy, ($C_2$-$C_4$)-Alkenyloxy, ($C_2$-$C_4$)-Alkinyloxy, Benzyloxy, Phenyloxy, ($C_3$-$C_8$)-Cycloalkyloxy, ($C_1$-$C_4$)-Alkylmercapto, Mono- oder Di-($C_1$-$C_4$)-Alkylamino, Cyano, Halogen und Nitro substituiert sind,

b) Phenyloxy, Phenylmercapto, Benzyloxy oder Benzylmercapto, welche jeweils unsubstituiert oder mit bis zu fünf gleichen oder verschiedenen Resten aus der Reihe ($C_1$-$C_4$)-Alkyl, ($C_2$-$C_4$)-Alkenyl, ($C_2$-$C_4$)-Alkinyl, Halogen, Cyano, Nitro, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, Mono- oder Di-($C_1$-$C_4$)-Alkylamino, Phenyloxy und Benzyloxy substituiert sind, oder Tri-($C_1$-$C_4$)-alkylsilylalkoxy bedeutet,

c) einen Rest der Formel -NR'R', worin R' Wasserstoff und/oder ($C_1$-$C_4$)-Alkyl bedeutet, Pyridino, Morpholino, Dimethylmorpholino, Hydrazino oder einen Rest der Formel

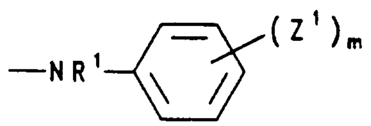

bedeutet, worin $R^1$ Wasserstoff oder ($C_1$-$C_4$)-Alkyl, die Reste $Z^1$ unabhängig voneinander Halogen, Nitro, ($C_1$-$C_4$)-Alkyl, ($C_1$-$C_4$)-Alkoxy oder Phenyloxy und m 0 bis 3 sind,

d) einen Rest der Formel $-O-N=CR^2R^2$ bedeutet, worin $R^2$ für ($C_1$-$C_4$)-Alkyl, steht oder die Reste $R^2$ gemeinsam mit dem sie verknüpfenden C-Atom für Cyclohexyliden oder Cyclopentyliden stehen,

e) einen Rest der Formel $-O-CR^3R^3-CO-R^4$, worin $R^3$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Phenyl, Benzyl oder ($C_1$-$C_4$)-Alkoxy steht und $R^4$ für ($C_1$-$C_4$)-Alkyl, ($C_2$-$C_4$)-Alkenyl, ($C_2$-$C_4$)-Alkinyl, Phenyl oder Benzyl steht,

f) einen Rest der Formel $-NH-N=CR^5R^5$, worin $R^5$ für gleiche oder verschiedene Reste aus der Reihe Wasserstoff, ($C_1$-$C_4$)-Alkyl oder Phenyl oder die beiden Reste $R^5$ gemeinsam mit dem sie verknüpfenden C-Atom Cyclohexyliden oder Cyclopentyliden bedeuten, oder

g) einen Rest der Formel $-O-CR^6R^6-CO-R^7$ bedeutet, worin $R^6$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Phenyl, Benzyl und ($C_1$-$C_4$)-Alkoxy stehen und $R^7$ eine der vorstehend für R unter a) bis f) angegebenen Bedeutungen hat.

**4.** Mittel gemäß einem der Ansprüche 1 bis 3, in welchem in Formel I

R Wasserstoff, ($C_1$-$C_4$)-Alkoxy, ($C_2$-$C_4$)-Alkenyloxy, ($C_2$-$C_4$)-Alkinyloxy, Benzyloxy, Phenyloxy, NR'R' mit R' = Wasserstoff und/oder ($C_1$-$C_4$)-Alkyl, Hydrazino oder $-O-CR^3R^3-CO-R^4$ mit $R^3$ = Wasserstoff und $R^4$ = ($C_1$-

$C_4$)-Alkoxy bedeutet.

5. Mittel gemäß einem der Ansprüche 1 bis 4, in welchem in Formel I

Z für gleiche oder verschiedene Reste aus der Reihe Halogen, ($C_1$-$C_4$)-Alkyl und ($C_1$-$C_4$)-Alkoxy steht und

n 0, 1 oder 2 ist.

6. Mittel gemäß einem der Ansprüche 1 bis 5, in welchem in Formel I

X für ein Sauerstoffatom steht.

7. Mittel gemäß einem der Ansprüche 1 bis 6, dadurch gekennzeichnet, daß es zusätzlich mindestens einen herbiziden Wirkstoff enthält.

8. Mittel gemäß Anspruch 7, dadurch gekennzeichnet, daß der herbizide Wirkstoff aus der Klasse der Carbamate, Thiocarbamate, Halogenacetanilide, substituierten Phenoxy-, Naphthoxy- und Phenoxyphenoxycarbonsäurederivate, Heteroaryloxyphenoxyalkancarbonsäurederivate, Cyclohexandionabkömmlinge, Imidazolinone und Sulfonylharnstoffe ausgewählt ist.

9. Verbindungen der Formel (I), wie sie in einem der Ansprüche 1 bis 6 definiert ist, und deren Salze, ausgenommen solche, worin

X = Sauerstoff bedeutet und

a)

R = OH, $OCH_3$, $OC_2H_5$, $OCH_2CH{=}CH{-}C_6H_5$, $OCH_2CH{=}CH{-}CH_3$ oder $OCH_2CH_2CH{=}CH{-}CH_3$ und
n = 0 bedeuten sowie

b)

R = einen Rest der Formel

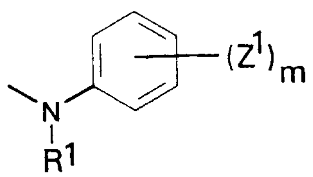

worin $R^1$ = H und m = 0 oder $R^1$ = H und $(Z^1)_m$ = 4-Cl sind, und
n = 0 bedeuten sowie

c)

R = $NH_2$ und
n = 0 bedeuten.

10. Verfahren zur Herstellung einer Verbindung gemäß Anspruch 9, dadurch gekennzeichnet, daß man eine Verbindung der Formel II

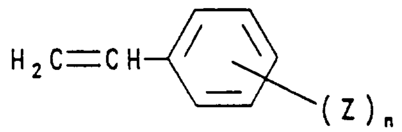

(II)

mit einer Verbindung der Formel III

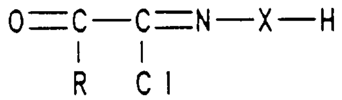

(III)

umsetzt, wobei in den Formeln II und III R, Z, n und X die bei Formel I definierten Bedeutungen haben, und die so erhaltene Verbindung der Formel I gegebenenfalls in ihr Salz überführt.

**11.** Verfahren zur Bekämpfung von unerwünschten Pflanzen in Nutzpflanzenkulturen, dadurch gekennzeichnet, daß man mindestens einen herbiziden Wirkstoff in Kombination mit mindestens einer gemäß einem der Ansprüche 1 bis 6 definierten Verbindung der Formel I auf Pflanzen, Pflanzenteile, Pflanzensamen oder die Anbaufläche appliziert.

**12.** Verfahren zur Bekämpfung von unerwünschten Pflanzen in Nutzpflanzenkulturen, dadurch gekennzeichnet, daß man mindestens einen herbiziden Wirkstoff in Kombination mit mindestens einer gemäß Anspruch 10 definierten Verbindung der Formel I auf Pflanzen, Pflanzenteile, Pflanzensamen oder die Anbaufläche appliziert.

**13.** Verwendung von einer gemäß einem der Ansprüche 1 bis 6 oder 9 definierten Verbindung der Formel I zum Schutz von Kulturpflanzen vor phytotoxischen Nebenwirkungen von Herbiziden.

**14.** Verfahren zu Herstellung eines Mittels gemäß einem der Ansprüche 1 bis 8, dadurch gekennzeichnet, daß man mindestens eine Verbindung, gegebenenfalls zusammen mit mindestens einem herbiziden Wirkstoff, und einem Formulierungshilfmittel in eine für den Pflanzenschutz geeignete Anwendungsform bringt.

**Patentansprüche für folgenden Vertragsstaat : ES**

**1.** Verwendung von Verbindungen der Formel I oder deren Salzen,

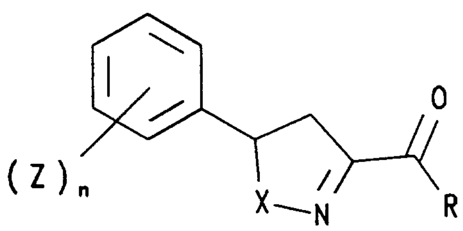

(I)

in welcher

X ein Sauerstoff- oder Schwefelatom bedeutet,
R

a) Hydroxy, Mercapto, $(C_1-C_5)$Alkoxy, $(C_2-C_5)$Alkenyloxy, $(C_2-C_5)$Alkinyloxy, $(C_1-C_5)$Alkylmercapto, $(C_2-C_5)$Alkenylmercapto, $(C_2-C_5)$Alkinylmercapto, $(C_3-C_7)$Cycloalkyloxy oder $(C_3-C_7)$Cycloalkylmercapto bedeutet, wobei die 8 letztgenannten Gruppen unsubstituiert oder mit einem oder mehreren gleichen oder verschiedenen Resten aus der Reihe $(C_6-C_{12})$Aryl, $(C_1-C_5)$Alkoxy, $(C_2-C_5)$Alkenyloxy, $(C_2-C_5)$Alkinyloxy, $(C_6-C_{12})$Aralkyloxy, $(C_6-C_{12})$Aryloxy, $(C_3-C_7)$Cycloalkyloxy, $(C_1-C_5)$Alkylmercapto, Mono- oder Di-$(C_1-C_5)$alkylamino, Cyano, Halogen und Nitro substituiert sind,

b) $(C_6-C_{12})$Aralkyloxy, $(C_6-C_{12})$Aryloxy, $(C_6-C_{12})$Aralkylmercapto oder $(C_6-C_{12})$Arylmercapto, welche jeweils unsubstituiert oder mit einem oder mehreren gleichen oder verschiedenen Resten aus der Reihe $(C_1-C_5)$Alkyl, $(C_2-C_5)$Alkenyl, $(C_2-C_5)$Alkinyl, Halogen, Cyano, Nitro, $(C_1-C_5)$Alkoxy, $(C_2-C_5)$Alkenyloxy, $(C_2-C_5)$Alkinyloxy, $(C_1-C_5)$Alkylmercapto, Mono- oder Di-$(C_1-C_5)$alkylamino, $(C_6-C_{12})$Aryloxy und $(C_6-C_{12})$Aroyloxy substituiert sind, oder Tri-$(C_1-C_5)$alkylsilyl-$(C_1-C_5)$alkoxy, $(C_6-C_{12})$Aryldi-$(C_1-C_5)$alkylsilyloxy, $(C_6-C_{12})$Aryl$(C_1-C_5)$alkyldi-$(C_1-C_5)$alkylsilyloxy, Di-$(C_6-C_{12})$aryl$(C_1-C_5)$alkylsilyloxy oder Di-[$(C_6-C_{12})$aryl$(C_1-C_5)$alkyl]-$(C_1-C_5)$alkylsilyloxy bedeutet,

c) einen Rest der Formel NR'R', worin die R' für gleiche oder verschiedene Reste aus der Gruppe Wasserstoff, $(C_1-C_5)$Alkyl, $(C_2-C_5)$Alkenyl, $(C_2-C_5)$Alkinyl und $(C_3-C_7)$Cycloalkyl steht, oder Pyridino, Morpholino, Di-$(C_1-C_5)$alkylmorpholino, Hydrazino oder einen Rest der Formel

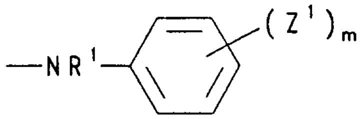

bedeutet,
worin $R^1$ Wasserstoff, $(C_1-C_5)$Alkyl, $(C_2-C_5)$Alkenyl oder $(C_2-C_5)$Alkinyl, die Reste $Z^1$ unabhängig voneinander Halogen, Nitro, $(C_1-C_5)$Alkyl, $(C_2-C_5)$Alkenyl, $(C_1-C_5)$Alkoxy oder $(C_6-C_{12})$Aryloxy und m eine ganze Zahl von 0 bis 5 sind,
d) einen Rest der Formel

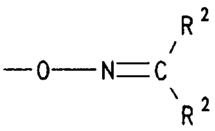

bedeutet, worin die Reste $R^2$ unabhängig voneinander $(C_1-C_5)$Alkyl oder gemeinsam mit dem sie verknüpfenden C-Atom $(C_3-C_7)$Cycloalkyliden bedeuten,
e) einen Rest der Formel -O-CR$^3$R$^3$-CO-R$^4$ bedeutet, worin $R^3$ für gleiche oder verschiedene Reste aus der Reihe Wasserstoff, $(C_1-C_5)$Alkyl, $(C_2-C_5)$Alkenyl, $(C_2-C_5)$Alkinyl, $(C_6-C_{12})$Aryl, $(C_6-C_{12})$Aryl $(C_1-C_5)$alkyl, $(C_1-C_5)$Alkoxy, $(C_2-C_5)$Alkenyloxy, $(C_2-C_5)$Alkinyloxy und $(C_6-C_{12})$Aryloxy steht und $R^4$ für Wasserstoff, $(C_1-C_5)$Alkyl, $(C_2-C_5)$Alkenyl, $(C_2-C_5)$Alkinyl, $(C_6-C_{12})$Aryl oder $(C_6-C_{12})$Aryl-$(C_1-C_5)$alkyl steht,
f) einen Rest der Formel

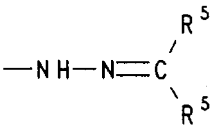

bedeutet, worin $R^5$ für gleiche oder verschiedene Reste aus der Reihe Wasserstoff, $(C_1-C_5)$Alkyl und $(C_6-C_{12})$Aryl steht oder die beiden Reste $R^5$ gemeinsam mit dem sie verknüpfenden C-Atom $(C_3-C_7)$Cycloalkyliden bedeuten, oder
g) einen Rest der Formel -O-CR$^6$R$^6$-CO-R$^7$ bedeutet, worin $R^6$ für gleiche oder verschiedene Reste aus der Reihe Wasserstoff, $(C_1-C_5)$Alkyl, $(C_2-C_5)$Alkenyl, $(C_2-C_5)$Alkinyl, $(C_6-C_{12})$Aryl, $(C_6-C_{12})$Aryl-$(C_1-C_5)$alkyl, $(C_1-C_5)$Alkoxy, $(C_2-C_5)$Alkenyloxy, $(C_2-C_5)$Alkinyloxy und $(C_6-C_{12})$Aryloxy steht und $R^7$ eine der vorstehend für R unter a) bis f) angegebenen Bedeutungen hat,

Z Halogen, Nitro, Cyano, $(C_1-C_4)$-Alkyl, $(C_1-C_4)$-Alkoxy, $(C_1-C_4)$-Alkylmercapto, wobei die Alkyl-, Alkoxy- und Alkylmercaptogruppen unabhängig voneinander jeweils unsubstituiert oder mit einem oder mehreren Halogenatomen substituiert sind, $(C_3-C_6)$-Cycloalkyl, das unsubstituiert oder mit vorzugsweise bis zu drei $(C_1-C_4)$-Alkyl substituiert ist, Amino, Hydroxymethyl, $(C_1-C_4)$-Alkylamino, Di-$(C_1-C_4)$-alkylamino, $(C_1-C_4)$-Alkoxymethyl, wobei die Alkyl- und Alkoxygruppen in den drei letztgenannten Resten unabhängig voneinander unsubstituiert oder mit vorzugsweise bis zu drei $(C_1-C_4)$-Alkyl substituiert sind, $(C_6-C_{12})$Aryl oder $(C_6-$

$C_{12}$)Aryloxy, wobei Aryl und Aryloxy unabhängig voneinander jeweils unsubstituiert oder ein- oder mehrfach gleichen oder verschiedenen Resten aus der Reihe Halogen und Trifluormethyl substituiert sind, bedeutet und

n eine ganze Zahl von 0 bis 5 ist,

zum Schutz von Kulturpflanzen vor phytotoxischen Nebenwirkungen von Herbiziden.

**2.** Verwendung gemäß Anspruch 1, wobei in Formel I

R

a) Hydroxy, Mercapto, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, ($C_2$-$C_4$)-Alkenylmercapto, ($C_2$-$C_4$)-Alkinylmercapto oder ($C_3$-$C_8$)-Cycloalkylmercapto bedeutet, wobei die 8 letztgenannten Gruppen unsubstituiert oder mit einem oder mehreren gleichen oder verschiedenen Resten aus der Reihe Aryl, ($C_1$-$C_4$)-Alkoxy, ($C_2$-$C_4$)-Alkenyloxy, ($C_2$-$C_4$)-Alkinyloxy, Aralkyloxy, Aryloxy, ($C_3$-$C_8$)-Cycloalkyloxy, ($C_1$-$C_4$)-Alkylmercapto, Mono- oder Di-($C_1$-$C_4$)-Alkylamino, Cyano, Halogen und Nitro substituiert sind,
b) Aryloxy, Arylmercapto, Aralkyloxy oder Aralkylmercapto, welche jeweils unsubstituiert oder mit einem oder mehreren gleichen oder verschiedenen Resten aus der Reihe ($C_1$-$C_4$)-Alkyl, ($C_2$-$C_4$)-Alkenyl, ($C_2$-$C_4$)-Alkinyl, Halogen, Cyano, Nitro, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, Mono- oder Di-($C_1$-$C_4$)-Alkylamino, Aryloxy und Aralkyloxy substituiert sind, oder Tri-($C_1$-$C_4$)-alkylsilylalkoxy bedeutet,
c) einen Rest der Formel -NR'R', worin R' Wasserstoff und/oder ($C_1$-$C_4$)-Alkyl bedeutet, Pyridino, Morpholino, Dimethylmorpholino, Hydrazino oder einen Rest der Formel

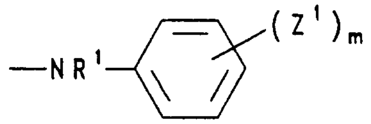

bedeutet, worin $R^1$ Wasserstoff oder ($C_1$-$C_4$)-Alkyl, die Reste $Z^1$ unabhängig voneinander Halogen, Nitro, ($C_1$-$C_4$)-Alkyl, ($C_1$-$C_4$)-Alkoxy oder Aryloxy und m 0 bis 3 sind,
d) einen Rest der Formel $-O-N=CR^2R^2$ bedeutet, worin $R^2$ für ($C_1$-$C_4$)-Alkyl steht oder die Reste $R^2$ gemeinsam mit dem sie verknüpfenden C-Atom für Cyclohexyliden oder Cyclopentyliden stehen,
e) einen Rest der Formel $-O-CR^3R^3-CO-R^4$, worin $R^3$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Aryl, Aralkyl oder ($C_1$-$C_4$)-Alkoxy steht und $R^4$ für ($C_1$-$C_4$)-Alkyl, ($C_2$-$C_4$)-Alkenyl, ($C_2$-$C_4$)-Alkinyl, Aryl oder Aralkyl steht,
f) einen Rest der Formel $-NH-N=CR^5R^5$, worin $R^5$ für gleiche oder verschiedene Reste aus der Reihe Wasserstoff, ($C_1$-$C_4$)-Alkyl oder Aryl oder die beiden Reste $R^5$ gemeinsam mit dem sie verknüpfenden C-Atom Cyclohexyliden oder Cyclopentyliden bedeuten, oder
g) einen Rest der Formel $-O-CR^6R^6-CO-R^7$ bedeutet, worin $R^6$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Aryl, Aralkyl und ($C_1$-$C_4$)-Alkoxy stehen und $R^7$ eine der vorstehend für R unter a) bis f) angegebenen Bedeutungen hat.

**3.** Verwendung gemäß Anspruch 1 oder 2, wobei in Formel I

R

a) Hydroxy, Mercapto, ($C_1$-$C_4$)-Alkoxy, ($C_2$-$C_4$)-Alkenyloxy, ($C_2$-$C_4$)-Alkinyloxy, ($C_1$-$C_4$)-Alkylmercapto, ($C_2$-$C_4$)-Alkenylmercapto, ($C_2$-$C_4$)-Alkinylmercapto oder ($C_3$-$C_8$)-Cycloalkylmercapto bedeutet, wobei die 8 letztgenannten Gruppen unsubstituiert oder mit bis zu drei gleichen oder verschiedenen Resten aus der Reihe Phenyl, ($C_1$-$C_4$)-Alkoxy, ($C_2$-$C_4$)-Alkenyloxy, ($C_2$-$C_4$)-Alkinyloxy, Benzyloxy, Phenyloxy, ($C_3$-$C_8$)-Cycloalkyloxy, ($C_1$-$C_4$)-Alkylmercapto, Mono- oder Di-($C_1$-$C_4$)-Alkylamino, Cyano, Halogen und Nitro substituiert sind,
b) Phenyloxy, Phenylmercapto, Benzyloxy oder Benzylmercapto, welche jeweils unsubstituiert oder mit bis zu fünf gleichen oder verschiedenen Resten aus der Reihe ($C_1$-$C_4$)-Alkyl, ($C_2$-$C_4$)-Alkenyl, ($C_2$-$C_4$)-Alkinyl, Halogen, Cyano, Nitro, ($C_1$-$C_4$)-Alkoxy, ($C_2$-$C_4$)-Alkenyloxy, ($C_2$-$C_4$)-Alkinyloxy, ($C_1$-$C_4$)-

Alkylmercapto, Mono- oder Di-($C_1$-$C_4$)-Alkylamino, Phenyloxy und Benzyloxy substituiert sind, oder Tri-($C_1$-$C_4$)-alkylsilylalkoxy bedeutet,

c) einen Rest der Formel -NR'R', worin R' Wasserstoff und/oder ($C_1$-$C_4$)-Alkyl bedeutet, Pyridino, Morpholino, Dimethylmorpholino, Hydrazino oder einen Rest der Formel

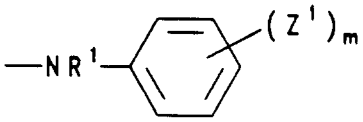

bedeutet, worin $R^1$ Wasserstoff oder ($C_1$-$C_4$)-Alkyl, die Reste $Z^1$ unabhängig voneinander Halogen, Nitro, ($C_1$-$C_4$)-Alkyl, ($C_1$-$C_4$)-Alkoxy oder Phenyloxy und m 0 bis 3 sind,

d) einen Rest der Formel -O-N=$CR^2R^2$ bedeutet, worin $R^2$ für ($C_1$-$C_4$)-Alkyl, steht oder die Reste $R^2$ gemeinsam mit dem sie verknüpfenden C-Atom für Cyclohexyliden oder Cyclopentyliden stehen,

e) einen Rest der Formel -O-$CR^3R^3$-CO-$R^4$, worin $R^3$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Phenyl, Benzyl oder ($C_1$-$C_4$)-Alkoxy steht und $R^4$ für ($C_1$-$C_4$)-Alkyl, ($C_2$-$C_4$)-Alkenyl, ($C_2$-$C_4$)-Alkinyl, Phenyl oder Benzyl steht,

f) einen Rest der Formel -NH-N=$CR^5R^5$, worin $R^5$ für gleiche oder verschiedene Reste aus der Reihe Wasserstoff, ($C_1$-$C_4$)-Alkyl oder Phenyl oder die beiden Reste $R^5$ gemeinsam mit dem sie verknüpfenden C-Atom Cyclohexyliden oder Cyclopentyliden bedeuten, oder

g) einen Rest der Formel -O-$CR^6R^6$-CO-$R^7$ bedeutet, worin $R^6$ für gleiche oder verschiedene Reste aus der Reihe Wasserstoff, ($C_1$-$C_4$)-Alkyl, ($C_2$-$C_4$)-Alkenyl, ($C_2$-$C_4$)-Alkinyl, Phenyl, Benzyl und ($C_1$-$C_4$)-Alkoxy stehen und $R^7$ eine der vorstehend für R unter a) bis f) angegebenen Bedeutungen hat.

**4.** Verwendung gemäß einem der Ansprüche 1 bis 3, wobei in Formel I

R Wasserstoff, ($C_1$-$C_4$)-Alkoxy, ($C_2$-$C_4$)-Alkenyloxy, ($C_2$-$C_4$)-Alkinyloxy, Benzyloxy, Phenyloxy, NR'R' mit R' = Wasserstoff und/oder ($C_1$-$C_4$)-Alkyl, Hydrazino oder -O-$CR^3R^3$-CO-$R^4$ mit $R^3$ = Wasserstoff und $R^4$ = ($C_1$-$C_4$)-Alkoxy bedeutet.

**5.** Verwendung gemäß einem der Ansprüche 1 bis 4, wobei in Formel I

Z für gleiche oder verschiedene Reste aus der Reihe Halogen, ($C_1$-$C_4$)-Alkyl und ($C_1$-$C_4$)-Alkoxy steht und

n 0, 1 oder 2 ist.

**6.** Verwendung gemäß einem der Ansprüche 1 bis 5, wobei in Formel I

X für ein Sauerstoffatom steht.

**7.** Verwendung gemäß einem der Ansprüche 1 bis 6, dadurch gekennzeichnet, daß man die Verbindung der Formel I oder deren Salze zusammen mit mindestens einem herbiziden Wirkstoff verwendet.

**8.** Verwendung gemäß Anspruch 7, dadurch gekennzeichnet, daß der herbizide Wirkstoff aus der Klasse der Carbamate, Thiocarbamate, Halogenacetanilide, substituierten Phenoxy-, Naphthoxy- und Phenoxy-phenoxycarbonsäurederivate, Heteroaryloxy-phenoxyalkancarbonsäurederivate Cyclohexandionabkömmlinge, Imidazolinone und Sulfonylharnstoffe ist.

**9.** Verwendung nach einem der Ansprüche 1 bis 8, ausgenommen solche, wobei in den Verbindungen der Formel (I)

X = Sauerstoff bedeutet und

a)

R = OH, $OCH_3$, $OC_2H_5$, $OCH_2CH=CH-C_6H_5$, $OCH_2CH=CH-CH_3$ oder $OCH_2CH_2CH=CH-CH_3$ und
n = 0 bedeuten sowie

b)

R = einen Rest der Formel

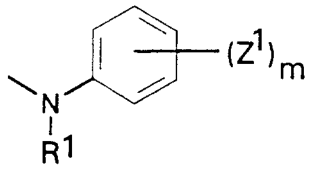

worin $R^1$ = H und m = 0 oder $R^1$ = H und $(Z^1)_m$ = 4-Cl sind, und

n = 0 bedeuten sowie

c)

R = $NH_2$ und

n = 0 bedeuten.

**10.** Verfahren zur Herstellung einer gemäß Anspruch 9 definierten Verbindung der Formel I, dadurch gekennzeichnet, daß man eine Verbindung der Formel II

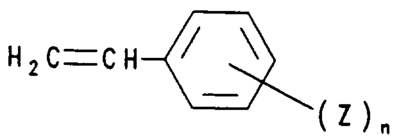

(II)

mit einer Verbindung der Formel III

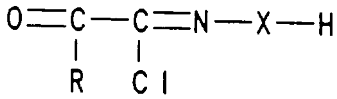

(III)

umsetzt, wobei in den Formeln II und III R, Z, n und X die bei Formel I definierten Bedeutungen haben, und die so erhaltene Verbindung der Formel I gegebenenfalls in ihr Salz überführt.

**11.** Verfahren zur Bekämpfung von unerwünschten Pflanzen in Nutzpflanzenkulturen, dadurch gekennzeichnet, daß man mindestens einen herbiziden Wirkstoff in Kombination mit mindestens einer gemäß einem der Ansprüche 1 bis 6 definierten Verbindung der Formel I auf Pflanzen, Pflanzenteile, Pflanzensamen oder die Anbaufläche appliziert.

**12.** Verfahren zur Bekämpfung von unerwünschten Pflanzen in Nutzpflanzenkulturen, dadurch gekennzeichnet, daß man mindestens einen herbiziden Wirkstoff in Kombination mit mindestens einer gemäß Anspruch 10 definierten Verbindung der Formel I auf Pflanzen, Pflanzenteile, Pflanzensamen oder die Anbaufläche appliziert.

**13.** Verfahren zu Herstellung eines kulturpflanzenschützenden Mittels, dadurch gekennzeichnet, daß man mindestens eine Verbindung, der Formel I oder deren Salze gemäß einem der Ansprüche 1 bis 8, gegebenenfalls zusammen mit mindestens einem herbiziden Wirkstoff, und einem Formulierungshilfmittel in eine für den Pflanzenschutz geeignete Anwendungsform bringt.

## Claims

**Claims for the following Contracting States : AT, BE, CH, LI, DE, DK, FR, GB, IT, NL, PT, SE**

**1.** A crop-plant-protecting composition which contains at least one compound of the formula I or a salt thereof,

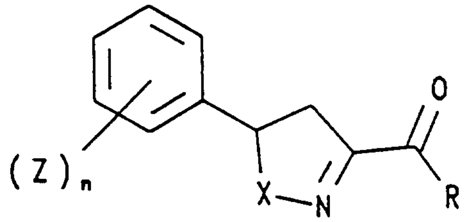

(I)

in which

X is an oxygen or sulfur atom,
R is

a) hydroxyl, mercapto, $(C_1\text{-}C_5)$alkoxy, $(C_2\text{-}C_5)$-alkenyloxy, $(C_2\text{-}C_5)$alkynyloxy, $(C_1\text{-}C_5)$-alkylmercapto, $(C_2\text{-}C_5)$alkenylmercapto, $(C_2\text{-}C_5)$-alkynylmercapto, $(C_3\text{-}C_7)$cycloalkyloxy or $(C_3\text{-}C_7)$cycloalkylmercapto, the last 8 groups mentioned being unsubstituted or substituted by one or more identical or different radicals selected from the group comprising $(C_6\text{-}C_{12})$aryl, $(C_1\text{-}C_5)$alkoxy, $(C_2\text{-}C_5)$alkenyloxy, $(C_2\text{-}C_5)$alkynyloxy, $(C_6\text{-}C_{12})$-aralkyloxy, $(C_6\text{-}C_{12})$aryloxy, $(C_3\text{-}C_7)$cycloalkyloxy, $(C_1\text{-}C_5)$alkylmercapto, mono- or di-$(C_1\text{-}C_5)$alkylamino, cyano, halogen and nitro,
b) $(C_6\text{-}C_{12})$aralkyloxy, $(C_6\text{-}C_{12})$aryloxy, $(C_6\text{-}C_{12})$-aralkylmercapto or $(C_6\text{-}C_{12})$ar-ylmercapto, each of which is unsubstituted or substituted by one or more identical or different radicals selected from the group comprising $(C_1\text{-}C_5)$alkyl, $(C_2\text{-}C_5)$alkenyl, $(C_2\text{-}C_5)$alkynyl, halogen, cyano, nitro, $(C_1\text{-}C_5)$-alkoxy, $(C_2\text{-}C_5)$alkenyloxy, $(C_2\text{-}C_5)$alkynyloxy, $(C_1\text{-}C_5)$alkylmercapto, mono- or di-$(C_1\text{-}C_5)$alkylamino, $(C_6\text{-}C_{12})$ar-yloxy and $(C_6\text{-}C_{12})$-aroyloxy, or is tri-$(C_1\text{-}C_5)$alkylsilyl-$(C_1\text{-}C_5)$-alkoxy, $(C_6\text{-}C_{12})$aryldi-$(C_1\text{-}C_5)$alkylsilyloxy, $(C_6\text{-}C_{12})$aryl$(C_1\text{-}C_5)$alk-yldi-$(C_1\text{-}C_5)$alkylsilyloxy, di-$(C_6\text{-}C_{12})$aryl-$(C_1\text{-}C_5)$alkylsilyloxy or di-[$(C_6\text{-}C_{12})$aryl$(C_1\text{-}C_5)$-alkyl]-$(C_1\text{-}C_5)$alkylsilyoxy,
c) a radical of the formula NR'R', R' being identical or different radicals selected from the group comprising hydrogen, $(C_1\text{-}C_5)$alkyl, $(C_2\text{-}C_5)$alkenyl, $(C_2\text{-}C_5)$alkynyl and $(C_3\text{-}C_7)$cycloalkyl, or is pyridino, morpholino, di-$(C_1\text{-}C_5)$alkylmorpholino, hydrazino or a radical of the formula

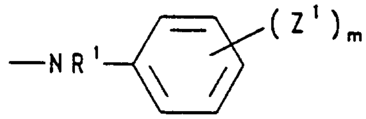

in which $R^1$ is hydrogen, $(C_1\text{-}C_5)$alkyl, $(C_2\text{-}C_5)$alkenyl or $(C_2\text{-}C_5)$alkynyl, the radicals $Z^1$ independently of one another are halogen, nitro, $(C_1\text{-}C_5)$alkyl, $(C_2\text{-}C_5)$alkenyl, $(C_1\text{-}C_5)$alkoxy or $(C_6\text{-}C_{12})$aryloxy, and m is an integer from 0 to 5,
d) a radical of the formula

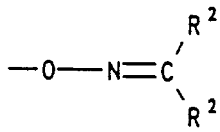

in which the radicals $R^2$ independently of one another are $(C_1\text{-}C_5)$alkyl or together with the carbon atom linking them are $(C_3\text{-}C_7)$cycloalkylidene,
e) a radical of the formula $-O\text{-}CR^3R^3\text{-}CO\text{-}R^4$ in which $R^3$ radicals are identical or different radicals

selected from the group comprising hydrogen, ($C_1$-$C_5$)alkyl, ($C_2$-$C_5$)alkenyl, ($C_2$-$C_5$)alkynyl, ($C_6$-$C_{12}$)aryl, ($C_6$-$C_{12}$)aryl($C_1$-$C_5$)alkyl, ($C_1$-$C_5$)alkoxy, ($C_2$-$C_5$)alkenyloxy, ($C_2$-$C_5$)alkynyloxy and ($C_6$-$C_{12}$)aryloxy, and $R^4$ is hydrogen, ($C_1$-$C_5$)alkyl, ($C_2$-$C_5$)alkenyl, ($C_2$-$C_5$)alkynyl, ($C_6$-$C_{12}$)aryl or ($C_6$-$C_{12}$)aryl-($C_1$-$C_5$)alkyl,

f) a radical of the formula

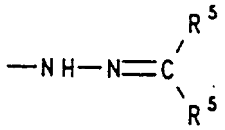

in which $R^5$ is identical or different radicals selected from the group comprising hydrogen, ($C_1$-$C_5$)alkyl and ($C_6$-$C_{12}$)aryl, or the two radicals $R^5$ together with the carbon atom linking them are ($C_3$-$C_7$)cycloalkylidene, or

g) a radical of the formula -O-$CR^6R^6$-CO-$R^7$ in which $R^6$ radicals are identical or different radicals selected from the group comprising hydrogen, ($C_1$-$C_5$)alkyl, ($C_2$-$C_5$)alkenyl, ($C_2$-$C_5$)alkynyl, ($C_6$-$C_{12}$)aryl, ($C_6$-$C_{12}$)aryl-($C_1$-$C_5$)alkyl, ($C_1$-$C_5$)alkoxy, ($C_2$-$C_5$)alkenyloxy, ($C_2$-$C_5$)alkynyloxy and ($C_6$-$C_{12}$)aryloxy, and $R^7$ has one of the meanings given above tor R under a) to f),

Z is halogen, nitro, cyano, ($C_1$-$C_4$)-alkyl, ($C_1$-$C_4$)-alkoxy, ($C_1$-$C_4$)-alkylmercapto, the alkyl, alkoxy and alkylmercapto groups independently of one another in each case being unsubstituted or substituted by one or more halogen atoms, or is ($C_3$-$C_6$)-cycloalkyl which is unsubstituted or substituted by preferably up to three ($C_1$-$C_4$)-alkyl radicals, amino, hydroxymethyl, ($C_1$-$C_4$)-alkylamino, di-($C_1$-$C_4$)-alkylamino, ($C_1$-$C_4$)-alkoxymethyl, the alkyl and alkoxy groups in the last three radicals mentioned independently of one another being unsubstituted or substituted by preferably up to three ($C_1$-$C_4$)-alkyl radicals, or ($C_6$-$C_{12}$)aryl or ($C_6$-$C_{12}$)aryloxy, aryl and aryloxy independently of one another in each case being unsubstituted or mono- or polysubstituted by identical or different radicals selected from the group comprising halogen and trifluoromethyl, and

n is an integer from 0 to 5,

and from 0.1 to 25% by weight, based on the composition, of surfactant.

**2.** The composition as claimed in claim 1, in which, in formula I,

R is

a) hydroxyl, mercapto, ($C_1$-$C_4$)-alkoxy, ($C_2$-$C_4$)-alkenyloxy, ($C_2$-$C_4$)-alkynyloxy, ($C_1$-$C_4$)-alkylmercapto, ($C_2$-$C_4$)-alkenylmercapto, ($C_2$-$C_4$)-alkynylmercapto or ($C_3$-$C_8$)-cycloalkylmercapto, the last 8 groups mentioned being unsubstituted or substituted by one or more identical or different radicals selected from the group comprising aryl, ($C_1$-$C_4$)-alkoxy, ($C_2$-$C_4$)-alkenyloxy, ($C_2$-$C_4$)-alkynyloxy, aralkyloxy, aryloxy, ($C_3$-$C_8$)-cycloalkyloxy, ($C_1$-$C_4$)-alkylmercapto, mono- or di-($C_1$-$C_4$)-alkylamino, cyano, halogen and nitro,

b) aryloxy, arylmercapto, aralkyloxy or aralkylmercapto, each of which is unsubstituted or substituted by one or more identical or different radicals selected from the group comprising ($C_1$-$C_4$)-alkyl, ($C_2$-$C_4$)-alkenyl, ($C_2$-$C_4$)-alkynyl, halogen, cyano, nitro, ($C_1$-$C_4$)-alkoxy, ($C_2$-$C_4$)-alkenyloxy, ($C_2$-$C_4$)-alkynyloxy, ($C_1$-$C_4$)-alkylmercapto, mono- or di-($C_1$-$C_4$)-alkylamino, aryloxy and aralkyloxy, or is tri-($C_1$-$C_4$)-alkylsilylalkoxy,

c) a radical of the formula -NR'R' in which R' is hydrogen and/or ($C_1$-$C_4$)-alkyl, or is pyridino, morpholino, dimethylmorpholino, hydrazino or a radical of the formula

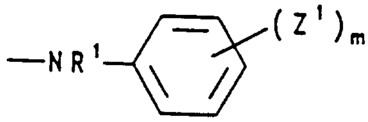

in which $R^1$ is hydrogen or $(C_1-C_4)$-alkyl, the radicals $Z^1$ independently of one another are halogen, nitro, $(C_1-C_4)$-alkyl, $(C_1-C_4)$-alkoxy or aryloxy, and m is 0 to 3,

d) a radical of the formula -O-N=$CR^2R^2$ in which $R^2$ is $(C_1-C_4)$-alkyl or the radicals $R^2$ together with the carbon atom linking them are cyclohexylidene or cyclopentylidene,

e) a radical of the formula -O-$CR^3R^3$-CO-$R^4$ in which $R^3$ radicals are identical or different radicals selected from the group comprising hydrogen, $(C_1-C_4)$-alkyl, $(C_2-C_4)$-alkenyl, $(C_2-C_4)$-alkynyl, aryl, aralkyl or $(C_1-C_4)$-alkoxy, and $R^4$ represents $(C_1-C_4)$-alkyl, $(C_2-C_4)$-alkenyl, $(C_2-C_4)$-alkynyl, aryl or aralkyl,

f) a radical of the formula -NH-N=$CR^5R^5$ in which $R^5$ radicals are identical or different radicals selected from the group comprising hydrogen, $(C_1-C_4)$-alkyl or aryl, or the two radicals $R^5$ together with the carbon atom linking them are cyclohexylidene or cyclopentylidene, or

g) a radical of the formula -O-$CR^6R^6$-CO-$R^7$ in which $R^6$ radicals are identical or different radicals selected from the group comprising hydrogen, $(C_1-C_4)$-alkyl, $(C_2-C_4)$-alkenyl, $(C_2-C_4)$-alkynyl, aryl, aralkyl and $(C_1-C_4)$-alkoxy, and $R^7$ has one of the meanings given above tor R under a) to f).

**3.** The composition as claimed in claim 1 or 2, in which, in formula I,

R is

a) hydroxyl, mercapto, $(C_1-C_4)$-alkoxy, $(C_2-C_4)$-alkenyloxy, $(C_2-C_4)$-alkynyloxy, $(C_1-C_4)$-alkylmercapto, $(C_2-C_4)$-alkenylmercapto, $(C_2-C_4)$-alkynylmercapto or $(C_3-C_8)$-cycloalkylmercapto, the last 8 groups mentioned being unsubstituted or substituted by up to three identical or different radicals selected from the group comprising phenyl, $(C_1-C_4)$-alkoxy, $(C_2-C_4)$-alkenyloxy, $(C_2-C_4)$-alkynyloxy, benzyloxy, phenyloxy, $(C_3-C_8)$-cycloalkyloxy, $(C_1-C_4)$-alkylmercapto, mono- or di-$(C_1-C_4)$-alkylamino, cyano, halogen and nitro,

b) phenyloxy, phenylmercapto, benzyloxy or benzylmercapto, each of which is unsubstituted or substituted by up to five identical or different radicals selected from the group comprising $(C_1-C_4)$-alkyl, $(C_2-C_4)$-alkenyl, $(C_2-C_4)$-alkynyl, halogen, cyano, nitro, $(C_1-C_4)$-alkoxy, $(C_2-C_4)$-alkenyloxy, $(C_2-C_4)$-alkynyloxy, $(C_1-C_4)$-alkylmercapto, mono- or di-$(C_1-C_4)$-alkylamino, phenyloxy and benzyloxy, or is tri-$(C_1-C_4)$-alkylsilylalkoxy,

c) a radical of the formula -NR'R' in which R' is hydrogen and/or $(C_1-C_4)$-alkyl, or is pyridino, morpholino, dimethylmorpholino, hydrazino or a radical of the formula

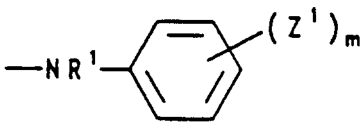

in which $R^1$ is hydrogen or $(C_1-C_4)$-alkyl, the radicals $Z^1$ independently of one another are halogen, nitro, $(C_1-C_4)$-alkyl, $(C_1-C_4)$-alkoxy or phenyloxy, and m is 0 to 3,

d) a radical of the formula -O-N=$CR^2R^2$ in which $R^2$ is $(C_1-C_4)$-alkyl or the radicals $R^2$ together with the carbon atom linking them are cyclohexylidene or cyclopentylidene,

e) a radical of the formula $-O-CR^3R^3-CO-R^4$ in which $R^3$ radicals are identical or different radicals selected from the group comprising hydrogen, $(C_1-C_4)$-alkyl, $(C_2-C_4)$-alkenyl, $(C_2-C_4)$-alkynyl, phenyl, benzyl or $(C_1-C_4)$-alkoxy, and $R^4$ is $(C_1-C_4)$-alkyl, $(C_2-C_4)$-alkenyl, $(C_2-C_4)$-alkynyl, phenyl or benzyl,

f) a radical of the formula $-NH-N=CR^5R^5$ in which $R^5$ radicals are identical or different radicals selected from the group comprising hydrogen, $(C_1-C_4)$-alkyl or phenyl, or the two radicals $R^5$ together with the carbon atom linking them are cyclohexylidene or cyclopentylidene, or

g) a radical of the formula $-O-CR^6R^6-CO-R^7$ in which $R^6$ radicals are identical or different radicals selected from the group comprising hydrogen, $(C_1-C_4)$-alkyl, $(C_2-C_4)$-alkenyl, $(C_2-C_4)$-alkynyl, phenyl, benzyl and $(C_1-C_4)$-alkoxy, and $R^7$ has one of the meanings given above for R under a) to f).

**4.** The composition as claimed in one of claims 1 to 3, in which, in formula I,

R is hydrogen, $(C_1-C_4)$-alkoxy, $(C_2-C_4)$-alkenyloxy, $(C_2-C_4)$-alkynyloxy, benzyloxy, phenyloxy, NR'R' where R' is hydrogen and/or $(C_1-C_4)$-alkyl, hydrazino or $-O-CR^3R^3-CO-R^4$ where $R^3$ is hydrogen and $R^4$ is $(C_1-C_4)$-alkoxy.

**5.** The composition as claimed in one of claims 1 to 4, in which, in formula I,

Z radicals are identical or different radicals selected from the group comprising halogen, $(C_1-C_4)$-alkyl and $(C_1-C_4)$-alkoxy and

n is 0, 1 or 2.

**6.** The composition as claimed in one of claims 1 to 5, in which, in formula I,

X is an oxygen atom.

**7.** The composition as claimed in one of claims 1 to 6, which additionally contains at least one herbicidal active substance.

**8.** The composition as claimed in claim 7, wherein the herbicidal active substance is selected from the class of the carbamates, thiocarbamates, haloacetanilides, substituted phenoxy-, naphthoxy- and phenoxyphenoxycarboxylic acid derivatives, heteroaryloxyphenoxyalkanecarboxylic acid derivatives, cyclohexanedione derivatives, imidazolinones and sulfonylureas.

**9.** A compound of the formula (I), as defined in one of claims 1 to 6, or a salt thereof, with the exception of that in which

X is oxygen and

a)

R is OH, $OCH_3$, $OC_2H_5$, $OCH_2CH=CH-C_6H_5$, $OCH_2CH=CH-CH_3$ or $OCH_2CH_2CH=CH-CH_3$ and
n is 0 and

b)

R is a radical of the formula

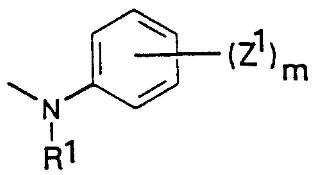

in which $R^1$ is H and m is 0 or $R^1$ is H and $(Z^1)_m$ is 4-Cl, and

n is 0 and

c)

R is $NH_2$ and

n is 0.

**10.** A process for the preparation of a compound as claimed in claim 9, which comprises reacting a compound of the formula II

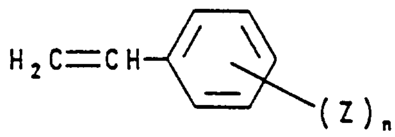

(II)

with a compound of the formula III

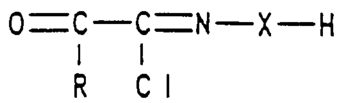

(III)

where in the formulae II and III R, Z, n and X have the meanings defined under formula I, and, if appropriate, converting the resulting compound of the formula I into its salt.

**11.** A method of controlling undesired plants in crops, which comprises applying at least one herbicidal active substance in combination with at least one compound of the formula I as defined in one of claims 1 to 6, to plants, parts of plants, seeds of plants or the area under cultivation.

**12.** A method of controlling undesired plants in crops, which comprises applying at least one herbicidal active substance in combination with at least one compound of the formula I as defined in claim 10, to plants, parts of plants, seeds of plants or the area under cultivation.

**13.** The use of a compound of the formula I as defined in one of claims 1 to 6 or 9, for protecting crop plants against phytotoxic secondary effects of herbicides.

**14.** A process for preparing a composition as claimed in one of claims 1 to 8, which comprises bringing at least one compound, optionally together with at least one herbicidal active substance, and a formulation auxiliary into a use form which is suitable for crop protection.

**Claims for the following Contracting State : ES**

**1.** The use of a compound of the formula I or a salt thereof,

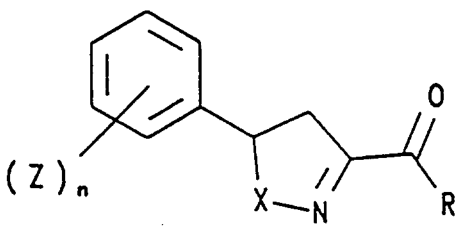

(I)

in which

X is an oxygen or sulfur atom,
R is

a) hydroxyl, mercapto, $(C_1-C_5)$alkoxy, $(C_2-C_5)$alkenyloxy, $(C_2-C_5)$alkynyloxy, $(C_1-C_5)$-alkylmercapto, $(C_2-C_5)$alkenylmercapto, $(C_2-C_5)$-alkynylmercapto, $(C_3-C_7)$cyclo-alkyloxy or $(C_3-C_7)$cycloalkylmercapto, the last 8 groups mentioned being unsubstituted er substituted by one or more identical or different radicals selected from the group comprising $(C_6-C_{12})$aryl, $(C_1-C_5)$alkoxy, $(C_2-C_5)$-alkenyloxy, $(C_2-C_5)$alkynyloxy, $(C_6-C_{12})$-aralkyloxy, $(C_6-C_{12})$aryloxy, $(C_3-C_7)$cyclo-alkyloxy, $(C_1-C_5)$alkylmercapto, mono- or di$(C_1-C_5)$alkylamino, cyano, halogen and nitro,
b) $(C_6-C_{12})$aralkyloxy, $(C_6-C_{12})$aryloxy, $(C_6-C_{12})$-aralkylmercapto er $(C_6-C_{12})$ar-ylmercapto, each of which is unsubstituted or substituted by one or more identical or different radicals selected from the group comprising $(C_1-C_5)$alkyl, $(C_2-C_5)$alkenyl, $(C_2-C_5)$-alkynyl, halogen, cyano, nitro, $(C_1-C_5)$-alkoxy, $(C_2-C_5)$alkenyloxy, $(C_2-C_5)$alkynyloxy, $(C_1-C_5)$alkylmercapto, mono- or di-$(C_1-C_5)$alkylamino, $(C_6-C_{12})$ar-yloxy and $(C_6-C_{12})$-aroyloxy, or is tri-$(C_1-C_5)$alkylsilyl-$(C_1-C_5)$-alkoxy, $(C_6-C_{12})$aryldi-$(C_1-C_5)$alkylsilyloxy, $(C_6-C_{12})$aryl$(C_1-C_5)$alk-yldi-$(C_1-C_5)$alkylsilyloxy, di-$(C_6-C_{12})$aryl-$(C_1-C_5)$alkylsilyloxy or di-[$(C_6-C_{12})$aryl$(C_1-C_5)$-alkyl]-$(C_1-C_5)$alkylsilyoxy,
c) a radical of the formula NR'R', R' being identical or different radicals selected from the group comprising hydrogen, $(C_1-C_5)$alkyl, $(C_2-C_5)$alkenyl, $(C_2-C_5)$alkynyl and $(C_3-C_7)$cycloalkyl, or is pyridino, morpholino, di-$(C_1-C_5)$alkylmorpholino, hydrazino or a radical of the formula

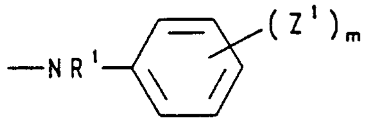

in which $R^1$ is hydrogen, $(C_1-C_5)$alkyl, $(C_2-C_5)$alkenyl or $(C_2-C_5)$alkynyl, the radicals $Z^1$ independently of one another are halogen, nitro, $(C_1-C_5)$alkyl, $(C_2-C_5)$alkenyl, $(C_1-C_5)$alkoxy or $(C_6-C_{12})$aryloxy, and m is an integer from 0 to 5,
d) a radical of the formula

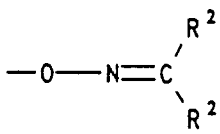

in which the radicals $R^2$ independently of one another are $(C_1-C_5)$alkyl or together with the carbon atom linking them are $(C_3-C_7)$cycloalkylidene,
e) a radical of the formula -O-$CR^3R^3$-CO-$R^4$ in which $R^3$ radicals are identical or different radicals selected from the group comprising hydrogen, $(C_1-C_5)$alkyl, $(C_2-C_5)$alkenyl, $(C_2-C_5)$alkynyl, $(C_6-C_{12})$aryl, $(C_6-C_{12})$aryl$(C_1-C_5)$alkyl, $(C_1-C_5)$alkoxy, $(C_2-C_5)$alkenyloxy, $(C_2-C_5)$alkynyloxy and $(C_6-C_{12})$aryloxy, and $R^4$ is hydrogen, $(C_1-C_5)$alkyl, $(C_2-C_5)$alkenyl, $(C_2-C_5)$alkynyl, $(C_6-C_{12})$aryl or $(C_6-C_{12})$aryl-$(C_1-C_5)$alkyl,
f) a radical of the formula

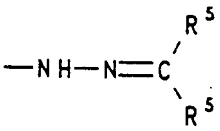

in which $R^5$ is identical or different radicals selected from the group comprising hydrogen, $(C_1-C_5)$alkyl and $(C_6-C_{12})$aryl, or the two radicals $R^5$ together with the carbon atom linking them are $(C_3-C_7)$cycloalkylidene, or

g) a radical of the formula -O-$CR^6R^6$-CO-$R^7$ in which $R^6$ radicals are identical or different radicals selected from the group comprising hydrogen, ($C_1$-$C_5$)alkyl, ($C_2$-$C_5$)alkenyl, ($C_2$-$C_5$)alkynyl, ($C_6$-$C_{12}$)aryl, ($C_6$-$C_{12}$)aryl-($C_1$-$C_5$)alkyl, ($C_1$-$C_5$)alkoxy, ($C_2$-$C_5$)alkenyloxy, ($C_2$-$C_5$)alkynyloxy and ($C_6$-$C_{12}$)aryloxy, and $R^7$ has one of the meanings given above tor R under a) to f),

Z is halogen, nitro, cyano, ($C_1$-$C_4$)-alkyl, ($C_1$-$C_4$)-alkoxy, ($C_1$-$C_4$)-alkylmercapto, the alkyl, alkoxy and alkylmercapto groups independently of one another in each case being unsubstituted or substituted by one or more halogen atoms, or is ($C_3$-$C_6$)-cycloalkyl which is unsubstituted or substituted by preferably up to three ($C_1$-$C_4$)-alkyl radicals, amino, hydroxymethyl, ($C_1$-$C_4$)-alkylamino, di-($C_1$-$C_4$)-alkylamino, ($C_1$-$C_4$)-alkoxymethyl, the alkyl and alkoxy groups in the last three radicals mentioned independently of one another being unsubstituted or substituted by preferably up to three ($C_1$-$C_4$)-alkyl radicals, or ($C_6$-$C_{12}$)aryl or ($C_6$-$C_{12}$)aryloxy, aryl and aryloxy independently of one another in each case being unsubstituted or mono- or polysubstituted by identical or different radicals selected from the group comprising halogen and trifluoromethyl, and

n is an integer from 0 to 5,

for protecting crop plants against phytotoxic secondary effects of herbicides.

**2.** The use as claimed in claim 1, where, in formula I,

R is

a) hydroxyl, mercapto, ($C_1$-$C_4$)-alkoxy, ($C_2$-$C_4$)-alkenyloxy, ($C_2$-$C_4$)-alkynyloxy, ($C_1$-$C_4$)-alkylmercapto, ($C_2$-$C_4$)-alkenylmercapto, ($C_2$-$C_4$)-alkynylmercapto or ($C_3$-$C_8$)-cycloalkylmercapto, the last 8 groups mentioned being unsubstituted or substituted by one or more identical or different radicals selected from the group comprising aryl, ($C_1$-$C_4$)-alkoxy, ($C_2$-$C_4$)-alkenyloxy, ($C_2$-$C_4$)-alkynyloxy, aralkyloxy, aryloxy, ($C_3$-$C_8$)-cycloalkyloxy, ($C_1$-$C_4$)-alkylmercapto, mono- or di-($C_1$-$C_4$)-alkylamino, cyano, halogen and nitro,

b) aryloxy, arylmercapto, aralkyloxy or aralkylmercapto, each of which is unsubstituted or substituted by one or more identical or different radicals selected from the group comprising ($C_1$-$C_4$)-alkyl, ($C_2$-$C_4$)-alkenyl, ($C_2$-$C_4$)-alkynyl, halogen, cyano, nitro, ($C_1$-$C_4$)-alkoxy, ($C_2$-$C_4$)-alkenyloxy, ($C_2$-$C_4$)-alkynyloxy, ($C_1$-$C_4$)-alkylmercapto, mono- or di-($C_1$-$C_4$)-alkylamino, aryloxy and aralkyloxy, or is tri-($C_1$-$C_4$)-alkylsilylalkoxy,

c) a radical of the formula -NR'R' in which R' is hydrogen and/or ($C_1$-$C_4$)-alkyl, or is pyridino, morpholino, dimethylmorpholino, hydrazino or a radical of the formula

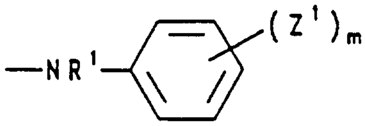

in which $R^1$ is hydrogen or ($C_1$-$C_4$)-alkyl, the radicals $Z^1$ independently of one another are halogen, nitro, ($C_1$-$C_4$)-alkyl, ($C_1$-$C_4$)-alkoxy or aryloxy, and m is 0 to 3,

d) a radical of the formula -O-N=$CR^2R^2$ in which $R^2$ is ($C_1$-$C_4$)-alkyl or the radicals $R^2$ together with the carbon atom linking them are cyclohexylidene or cyclopentylidene,

e) a radical of the formula -O-$CR^3R^3$-CO-$R^4$ in which $R^3$ radicals are identical or different radicals selected from the group comprising hydrogen, ($C_1$-$C_4$)-alkyl, ($C_2$-$C_4$)-alkenyl, ($C_2$-$C_4$)-alkynyl, aryl, aralkyl or ($C_1$-$C_4$)-alkoxy, and $R^4$ represents ($C_1$-$C_4$)-alkyl, ($C_2$-$C_4$)-alkenyl, ($C_2$-$C_4$)-alkynyl, aryl or aralkyl,

f) a radical of the formula -NH-N=$CR^5R^5$ in which $R^5$ radicals are identical or different radicals selected from the group comprising hydrogen, ($C_1$-$C_4$)-alkyl or aryl, or the two radicals $R^5$ together with the carbon atom linking them are cyclohexylidene or cyclopentylidene, or

g) a radical off the formula -O-$CR^6R^6$-CO-$R^7$ in which $R^6$ radicals are identical or different radicals selected from the group comprising hydrogen, ($C_1$-$C_4$)-alkyl, ($C_2$-$C_4$)-alkenyl, ($C_2$-$C_4$)-alkynyl, aryl, aralkyl and ($C_1$-$C_4$)-alkoxy, and $R^7$ has one of the meanings given above tor R under a) to f).

**3.** The use as claimed in claim 1 or 2, where, in formula I,

R is

a) hydroxyl, mercapto, ($C_1$-$C_4$)-alkoxy, ($C_2$-$C_4$)-alkenyloxy, ($C_2$-$C_4$)-alkynyloxy, ($C_1$-$C_4$)-alkylmercapto, ($C_2$-$C_4$)-alkenylmercapto, ($C_2$-$C_4$)-alkynylmercapto or ($C_3$-$C_8$)-cycloalkylmercapto, the last 8 groups mentioned being unsubstituted or substituted by up to three identical or different radicals selected from the group comprising phenyl, ($C_1$-$C_4$)-alkoxy, ($C_2$-$C_4$)-alkenyloxy, ($C_2$-$C_4$)-alkynyloxy, benzyloxy, phenyloxy, ($C_3$-$C_8$)-cycloalkyloxy, ($C_1$-$C_4$)-alkylmercapto, mono- or di-($C_1$-$C_4$)-alkylamino, cyano, halogen and nitro,

b) phenyloxy, phenylmercapto, benzyloxy or benzylmercapto, each of which is unsubstituted or substituted by up to five identical or different radicals selected from the group comprising ($C_1$-$C_4$)-alkyl, ($C_2$-$C_4$)-alkenyl, ($C_2$-$C_4$)-alkynyl, halogen, cyano, nitro, ($C_1$-$C_4$)-alkoxy, ($C_2$-$C_4$)-alkenyloxy, ($C_2$-$C_4$)-alkynyloxy, ($C_1$-$C_4$)-alkylmercapto, mono- or di-($C_1$-$C_4$)-alkylamino, phenyloxy and benzyloxy, or is tri-($C_1$-$C_4$)-alkylsilylalkoxy,

c) a radical of the formula -NR'R' in which R' is hydrogen and/or ($C_1$-$C_4$)-alkyl, or is pyridino, morpholino, dimethylmorpholino, hydrazino or a radical of the formula

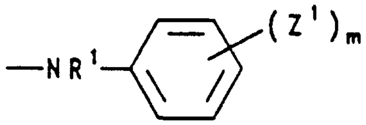

in which $R^1$ is hydrogen or ($C_1$-$C_4$)-alkyl, the radicals $Z^1$ independently of one another are halogen, nitro, ($C_1$-$C_4$)-alkyl, ($C_1$-$C_4$)-alkoxy or phenyloxy, and m is 0 to 3,

d) a radical of the formula -O-N=$CR^2R^2$ in which $R^2$ is ($C_1$-$C_4$)-alkyl or the radicals $R^2$ together with the carbon atom linking them are cyclohexylidene or cyclopentylidene,

e) a radical of the formula -O-$CR^3R^3$-CO-$R^4$ in which $R^3$ radicals are identical or different radicals selected from the group comprising hydrogen, ($C_1$-$C_4$)-alkyl, ($C_2$-$C_4$)-alkenyl, ($C_2$-$C_4$)-alkynyl, phenyl, benzyl or ($C_1$-$C_4$)-alkoxy, and $R^4$ is ($C_1$-$C_4$)-alkyl, ($C_2$-$C_4$)-alkenyl, ($C_2$-$C_4$)-alkynyl, phenyl or benzyl,

f) a radical of the formula -NH-N=$CR^5R^5$ in which $R^5$ radicals are identical or different radicals selected from the group comprising hydrogen, ($C_1$-$C_4$)-alkyl or phenyl, or the two radicals $R^5$ together with the carbon atom linking them are cyclohexylidene or cyclopentylidene, or

g) a radical of the formula -O-$CR^6R^6$-CO-$R^7$ in which $R^6$ radicals are identical or different radicals selected from the group comprising hydrogen, ($C_1$-$C_4$)-alkyl, ($C_2$-$C_4$)-alkenyl, ($C_2$-$C_4$)-alkynyl, phenyl, benzyl and ($C_1$-$C_4$)-alkoxy, and $R^7$ has one of the meanings given above tor R under a) to f).

**4.** The use as claimed in one of claims 1 to 3, where, in formula I,

R is hydrogen, ($C_1$-$C_4$)-alkoxy, ($C_2$-$C_4$)-alkenyloxy, ($C_2$-$C_4$)-alkynyloxy, benzyloxy, phenyloxy, NR'R' where R' is hydrogen and/or ($C_1$-$C_4$)-alkyl, hydrazino or -O-$CR^3R^3$-CO-$R^4$ where $R^3$ is hydrogen and $R^4$ is ($C_1$-$C_4$)-alkoxy.

**5.** The use as claimed in one of claims 1 to 4, where, in formula I,

Z radicals are identical or different radicals selected from the group comprising halogen, ($C_1$-$C_4$)-alkyl and ($C_1$-$C_4$)-alkoxy and

n is 0, 1 or 2.

6. The use as claimed in one of claims 1 to 5, where, in formula I,

X is an oxygen atom.

7. The use as claimed in one of claims 1 to 6, wherein the compound of the formula I or a salt thereof is used together with at least one herbicidal active substance.

8. The use as claimed in claim 7, wherein the herbicidal active substance is selected from the class of the carbamates, thiocarbamates, haloacetanilides, substituted phenoxy-, naphthoxy- and phenoxyphenoxycarboxylic acid derivatives, heteroaryloxyphenoxyalkanecarboxylic acid derivatives, cyclohexanedione derivatives, imidazolinones and sulfonylureas.

9. The use as claimed in one of claims 1 to 8, with the exception of that where, in the compounds of the formula (I),

X is oxygen and

a)

R is OH, $OCH_3$, $OC_2H_5$, $OCH_2CH=CH-C_6H_5$, $OCH_2CH=CH-CH_3$ or $OCH_2CH_2CH=CH-CH_3$ and
n is 0 and

b)

R is a radical of the formula

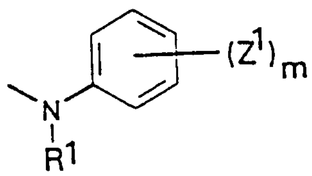

in which $R^1$ is H and m is 0 or $R^1$ is H and $(Z^1)_m$ is 4-Cl, and
n is 0 and

c)

R is $NH_2$ and
n is 0.

10. A process tor the preparation of a compound of the formula I as defined in claim 9, which comprises reacting a compound of the formula II

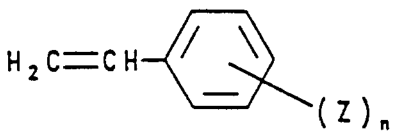

(II)

with a compound of the formula III

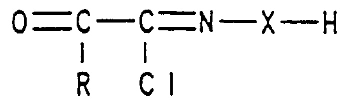

(III)

where in the formulae II and III R, Z, n and X have the meanings defined under formula I, and, if appropriate, converting the resulting compound of the formula I into its salt.

**11.** A method of controlling undesired plants in crops, which comprises applying at least one herbicidal active substance in combination with at least one compound of the formula I as defined in one of claims 1 to 6, to plants, parts of plants, seeds of plants or the area under cultivation.

**12.** A method of controlling undesired plants in crops, which comprises applying at least one herbicidal active substance in combination with at least one compound of the formula I as defined in claim 10, to plants, parts of plants, seeds of plants or the area under cultivation.

**13.** A process tor preparing a crop-plant-protecting composition, which comprises bringing at least one compound of the formula I or a salt thereof as claimed in one of claims 1 to 8, optionally together with at least one herbicidal active substance, and a formulation auxiliary into a use form which is suitable tor crop protection.

**Revendications**

**Revendications pour les Etats contractants suivants : AT, BE, CH, LI, DE, DK, FR, GB, IT, NL, PT, SE**

**1.** Agent de protection de cultures végétales contenant au moins un composé de formule I ou son sel

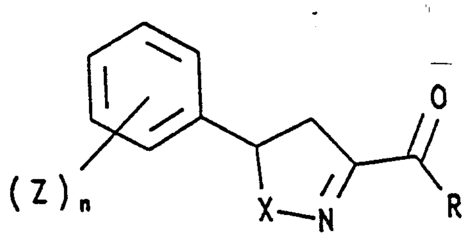

(I)

dans laquelle

X représente un atome d'oxygène ou de soufre,

R)

a) représente des groupes hydroxy, mercapto, alkoxy en $C_1$-$C_5$, alcényloxy en $C_2$-$C_5$, alcynyloxy en $C_2$-$C_5$, (alkyl en $C_1$-$C_5$)mercapto, (alcényl en $C_2$-$C_5$)mercapto, (alcynyl en $C_2$-$C_5$)-mercapto, cycloalkoxy en $C_3$-$C_7$ ou (cycloalkyl en $C_3$-$C_7$)mercapto, les 8 derniers groupes pouvant être non substitués ou substitués par un ou plusieurs restes identiques ou différents pris dans le groupe des restes aryle en $C_6$-$C_{12}$, alkoxy en $C_1$-$C_5$, alcényloxy $C_2$-$C_5$, alcynyloxy en $C_2$-$C_5$, aralkyloxy en $C_6$-$C_{12}$, aryloxy en $C_6$-$C_{12}$, cycloalkoxy en $C_3$-$C_7$, (alkyl en $C_1$-$C_5$)mercapto, mono- ou di(alkyl en $C_1$-$C_5$)amino, cyano, halogène et nitro,

b) aralkyloxy en $C_6$-$C_{12}$, aryloxy en $C_6$-$C_{12}$, (aralkyl en $C_6$-$C_{12}$)mercapto ou (aryl en $C_6$-$C_{12}$)mercapto, chacun étant non substitué ou substitué par un ou plusieurs restes identiques au différents pris dans le groupe des restes alkyle en $C_1$-$C_5$, alcényle en $C_2$-$C_5$, alcynyle en $C_2$-$C_5$, halogène, cyano, nitro, alkoxy en $C_1$-$C_5$, alcényloxy en $C_2$-$C_5$, alcynyloxy en $C_2$-$C_5$, (alkyl en $C_1$-$C_5$)mercapto, mono- ou di-(alkyl en $C_1$-$C_5$)amino, aryloxy en $C_6$-$C_{12}$ et aroyloxy en $C_6$-$C_{12}$, ou représente des groupes tri(alkyl en $C_1$-$C_5$)-silyl-(alkoxy en $C_1$-$C_5$), (aryl en $C_6$-$C_{12}$)-di-(alkyl en $C_1$-$C_5$)-silyloxy, (aryl en $C_6$-$C_{12}$)-(alkyl en $C_1$-$C_5$)-di-(alkyl en $C_1$-$C_5$)silyloxy, di-(aryl en $C_6$-$C_{12}$)-(alkyl en $C_1$-$C_5$)silyloxy ou di-[(aryl en $C_6$-$C_{12}$)-(alkyl en $C_1$-$C_5$)]-(alkyl en $C_1$-$C_5$)-silyloxy,

c) un reste de formule -NR'R', où R' représente des restes identiques au différents pris dans le groupe

comportant des atomes d'hydrogène, des groupes alkyle en $C_1$-$C_5$, alcényle en $C_2$-$C_5$, alcynyle en $C_2$-$C_5$ et cycloalkyle en $C_3$-$C_7$, ou des groupes pyridino, morpholino, di-(alkyl en $C_1$-$C_5$)morpholino, hydrazino ou un reste de formule

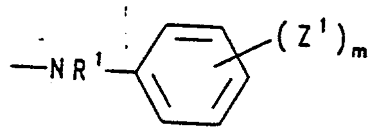

où $R^1$ représente un atome d'hydrogène, des groupes alkyle en $C_1$-$C_5$, alcényle en $C_2$-$C_5$ ou alcynyle en $C_2$-$C_5$, les restes $Z^1$ représentent, indépendamment les uns des autres, des atomes d'halogènes, des groupes nitro, alkyle en $C_1$-$C_5$, alcényle en $C_2$-$C_5$, alkoxy en $C_1$-$C_5$ ou aryloxy en $C_6$-$C_{12}$ et m est un entier de 0 à 5,

d) un reste de formule

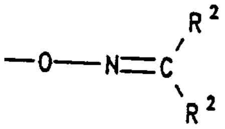

où les restes $R^2$ représentent indépendamment l'un de l'autre, des groupes alkyle en $C_1$-$C_5$ ou ensemble avec l'atome de carbone les reliant un groupe cycloalkylidène en $C_3$-$C_7$,

e) un reste de formule -O-$CR^3R^3$-CO-$R^4$, où $R^3$ représente des restes identiques ou différents pris dans le groupe comportant des atomes d'hydrogène, des groupes alkyle en $C_1$-$C_5$, alcényle en $C_2$-$C_5$, alcynyle en $C_2$-$C_5$, aryle en $C_6$-$C_{12}$, (aryl en $C_6$-$C_{12}$)-alkyle en $C_1$-$C_5$, alkoxy en $C_1$-$C_5$, alcényloxy en $C_2$-$C_5$, alcynyloxy en $C_2$-$C_5$ et aryloxy en $C_6$-$C_{12}$ et $R^4$ représente un atome d'hydrogène, des groupes alkyle en $C_1$-$C_5$, alcényle en $C_2$-$C_5$, alcynyle en $C_2$-$C_5$, aryle en $C_6$-$C_{12}$ ou aryl en $C_6$-$C_{12}$-alkyle en $C_1$-$C_5$,

f) un reste de formule

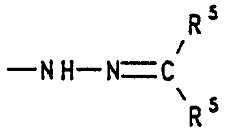

où les restes $R^5$ représentent des restes identiques ou différents pris dans le groupe comportant des atomes d'hydrogène, des restes alkyle en $C_1$-$C_5$ ou aryle en $C_6$-$C_{12}$ ou les deux restes $R^5$ ensemble avec l'atome de carbone les reliant, représentent un groupe cycloalkylidène en $C_3$-$C_7$, ou

g) un reste de formule -O-$CR^6R^6$-CO-$R^7$, où $R^6$ représente des restes identiques ou différents pris dans le groupe comportant des atomes d'hydrogène, des groupes alkyle en $C_1$-$C_5$, alcényle en $C_2$-$C_5$, alcynyle en $C_2$-$C_5$, aryle en $C_6$-$C_{12}$, (aryl en $C_6$-$C_{12}$)-alkyle en $C_1$-$C_5$, alkoxy en $C_1$-$C_5$, alcényloxy en $C_2$-$C_5$, alcynyloxy en $C_2$-$C_5$ et aryloxy en $C_6$-$C_{12}$ et $R^7$ possède l'une des significations données ci-dessus pour R aux points a) à f),

Z représente des atomes d'halogène, des groupes nitro, cyano, alkyle en $C_1$-$C_4$, alkoxy en $C_1$-$C_4$, (alkyl en $C_1$-$C_4$)mercapto, les groupes alkyle, alkoxy et alkylmercapto sont chacun, indépendamment l'un de l'autre, non substitués ou substitués par un ou plusieurs atomes d'halogène, un groupe cycloalkyle en $C_3$-$C_6$, lequel est non substitué ou substitué de préférence par jusqu'a trois groupes alkyle en $C_1$-$C_4$, représente des groupes amino, hydroxyméthyle, (alkyl en $C_1$-$C_4$)-amino, di-(alkyl en $C_1$-$C_4$)amino, (alkoxy en $C_1$-$C_4$)-méthyle, les groupes alkyle et alkoxy dans les trois derniers restes cités sont chacun, indépendamment les uns des autres, non substitué ou substitué par, de préférence, jusqu'à trois groupes alkyle en $C_1$-$C_4$, représente aryle en $C_6$-$C_{12}$ ou aryloxy en $C_6$-$C_{12}$, aryle et aryloxy étant chacun, indépendamment l'un de l'autre,

non substitué bu substitué une ou plusieurs fois, de préférence jusqu'à 5 fois, par des restes identiques ou différents pris parmi les restes halogène et trifluorométhyle, et

n est un entier de 0 à 5,

et de 0,1 à 25% en masse, par rapport à l'agent, d'un agent de surface.

**2.** Agent selon la revendication 1, où dans la formule I

R

a) représente des groupes hydroxy, mercapto, alkoxy en $C_1$-$C_4$, alcényloxy en $C_2$-$C_4$, alcynyloxy en $C_2$-$C_4$, (alkyl en $C_1$-$C_4$)mercapto, (alcényl en $C_2$-$C_4$)mercapto, (alcynyl en $C_2$-$C_4$)mercapto ou (cycloalkyl en $C_3$-$C_8$)-mercapto, les 8 derniers groupes pouvant être non substitués ou substitués par un ou plusieurs restes identiques ou différents pris dans le groupe des restes aryle, alkoxy en $C_1$-$C_4$, alcényloxy $C_2$-$C_4$, alcynyloxy en $C_2$-$C_4$, aralkyloxy, aryloxy, cycloalkoxy en $C_3$-$C_8$, (alkyl en $C_1$-$C_4$)mercapto, mono- ou di(alkyl en $C_1$-$C_4$)-amino, cyano, halogène et nitro,

b) aryloxy, arylmercapto, aralkyloxy ou aralkylmercapto, chacun étant non substitué ou substitué par un ou plusieurs restes identiques ou différents pris dans le groupe des restes alkyle en $C_1$-$C_4$, alcényle en $C_2$-$C_4$, alcynyle en $C_2$-$C_4$, halogène, cyano, nitro, alkoxy en $C_1$-$C_4$, alcényloxy en $C_2$-$C_4$, alcynyloxy en $C_2$-$C_4$, (alkyl en $C_1$-$C_4$)mercapto, mono- ou di-(alkyl en $C_1$-$C_4$)amino, aryloxy ou aralkyloxy, ou représente un groupe tri(alkyl en $C_1$-$C_4$)-silylalkoxy,

c) un reste de formule -NR'R', où R' représente un atome d'hydrogène et/ou un groupe alkyle en $C_1$-$C_4$, des groupes pyridino, morpholino, diméthylmorpholino, hydrazino ou un reste de formule

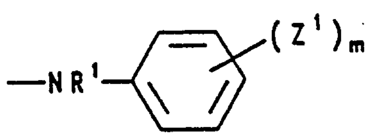

où $R^1$ représente un atome d'hydrogène ou un groupe alkyle en $C_1$-$C_4$, les restes $Z^1$ représentent, indépendamment les uns des autres, des atomes d'halogènes, des groupes nitro, alkyle en $C_1$-$C_4$, alkoxy en $C_1$-$C_4$ ou aryloxy et m est un entier de 0 à 3,

d) un reste de formule -O-N=$CR^2R^2$, où $R^2$ représente un groupe alkyle en $C_1$-$C_4$ ou les restes $R^2$ représentent ensemble avec l'atome de carbone qui les relie un groupe cyclohexylidène ou cyclopentylidène,

e) un reste de formule -O-$CR^3R^3$-CO-$R^4$, où $R^3$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, aryle, aralkyle ou alkoxy en $C_1$-$C_4$ et $R^4$ représente des groupes alkyle en $C_1$-$C_4$, alcényle en $C_2$-$C_4$, alcynyle en $C_2$-$C_4$, aryle ou aralkyle,

f) un reste de formule -NH-N=$CR^5R^5$, où $R^5$ représente des restes identiques ou différents pris dans le groupe comportant des atomes d'hydrogène, des groupes alkyle en $C_1$-$C_4$ ou aryle ou les deux restes $R^5$ représentent ensemble avec l'atome de carbone qui les relie un groupe cyclohexylidène ou cyclopentylidène, ou

g) un reste de formule -O-$CR^6R^6$-CO-$R^7$, où $R^6$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, aryle, aralkyle et alkoxy en $C_1$-$C_4$ et $R^7$ possède l'une des significations données pour R aux points a) à f).

**3.** Agent selon la revendication 1 ou 2, où dans la formule I

R

a) représente des groupes hydroxy, mercapto, alkoxy en $C_1$-$C_4$, alcényloxy en $C_2$-$C_4$, alcynyloxy en $C_2$-$C_4$, (alkyl en $C_1$-$C_4$)mercapto, (alcényl en $C_2$-$C_4$)mercapto, (alcynyl en $C_2$-$C_4$)mercapto ou (cycloalkyl en $C_3$-$C_8$)-mercapto, les 8 derniers groupes pouvant être non substitués ou substitués par jusqu'à trois restes identiques ou différents pris dans le groupe des restes phényle, alkoxy en $C_1$-$C_4$, alcényloxy $C_2$-$C_4$, alcynyloxy en $C_2$-$C_4$, benzyloxy, phényloxy, cycloalkoxy en $C_3$-$C_8$, (alkyl en $C_1$-$C_4$)mercapto, mono- ou di(alkyl en $C_1$-$C_4$)-amino, cyano, halogène et nitro,

b) phényloxy, phénylmercapto, benzyloxy ou benzylmercapto, chacun étant non substitué ou substitué par jusqu'à cinq restes identiques ou différents pris dans le groupe des restes alkyle en $C_1$-$C_4$, alcényle en $C_2$-$C_4$, alcynyle en $C_2$-$C_4$, halogène, cyano, nitro, alkoxy en $C_1$-$C_4$, alcényloxy en $C_2$-$C_4$, alcynyloxy en $C_2$-$C_4$, (alkyl en $C_1$-$C_4$)mercapto, mono- ou di-(alkyl en $C_1$-$C_4$)amino, phényloxy et benzyloxy, ou représente un groupe tri(alkyl en $C_1$-$C_4$)-silylalkoxy,

c) un reste de formule -NR'R', où R' représente un atome d'hydrogène et/ou un groupe alkyle en $C_1$-$C_4$, des groupes pyridino, morpholino, diméthylmorpholino, hydrazino ou un reste de formule

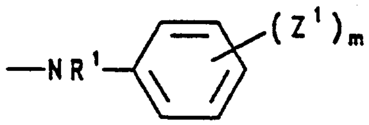

où $R^1$ représente un atome d'hydrogène ou un groupe alkyle en $C_1$-$C_4$, les restes $Z^1$ représentent, indépendamment les uns des autres, des atomes d'halogènes, des groupes nitro, alkyle en $C_1$-$C_4$, alkoxy en $C_1$-$C_4$ ou phényloxy et m est un entier de 0 à 3,

d) un reste de formule $-O-N=CR^2R^2$, où $R^2$ représente un groupe alkyle en $C_1$-$C_4$ ou les restes $R^2$ représentent ensemble avec l'atome de carbone qui les relie un groupe cyclohexylidène ou cyclopentylidène,

e) un reste de formule $-O-CR^3R^3-CO-R^4$, où $R^3$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, phényle, benzyle ou alkoxy en $C_1$-$C_4$ et $R^4$ représente des groupes alkyle en $C_1$-$C_4$, alcényle en $C_2$-$C_4$, alcynyle en $C_2$-$C_4$, phényle ou benzyle,

f) un reste de formule $-NH-N=CR^5R^5$, où $R^5$ représente des restes identiques ou différents pris dans le groupe comportant des atomes d'hydrogène, des groupes alkyle en $C_1$-$C_4$ ou phényle ou les deux restes $R^5$ représentent ensemble avec l'atome de carbone qui les relie un groupe cyclohexylidène ou cyclopentylidène, ou

g) un reste de formule $-O-CR^6R^6-CO-R^7$, où $R^6$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, phényle, benzyle et alkoxy en $C_1$-$C_4$ et $R^7$ possède l'une des significations données pour R aux points a) à f).

**4.** Agent selon l'une des revendications 1 à 3, où dans la formule I

R représente un atome d'hydrogène, des groupes alkoxy en $C_1$-$C_4$, alcényloxy en $C_2$-$C_4$, alcynyloxy en $C_2$-$C_4$, , phényloxy, NR'R' avec R' = hydrogène et/ou alkyle en $C_1$-$C_4$, hydrazino ou $-O-CR^3R^3-CO-R^4$ avec $R^3$ = hydrogène et $R^4$ = alkoxy en $C_1$-$C_4$.

**5.** Agent selon l'une des revendications 1 à 4, où dans la formule I

Z représente des restes identiques ou différents pris dans le groupe comportant des atomes d'halogènes, des groupes alkyle en $C_1$-$C_4$ ou alkoxy en $C_1$-$C_4$ et
n vaut 0, 1 ou 2.

**6.** Agent selon l'une des revendications 1 à 5, où dans la formule I

X représente un atome d'oxygène.

**7.** Agent selon l'une des revendications 1 à 6, caractérisé en ce qu'il contient de plus au moins une substance active herbicide.

**8.** Agent selon la revendication 7, caractérisé en ce que la substance active herbicide est prise dans la classe des carbamates, des thiocarbamates, des halogénoacétanilides, des dérives substitués des acides phénoxy-, naphtoxy- et phénoxy-phénoxycarboxyliques, des dérivés d'acide hétéroaryloxyphénoxyalcane carboxylique, des dérivés de cyclohexane dione, des imidazolinones et des sulfonylurées.

**9.** Composés de formule (I), tels que définis aux revendications 1 à 6, et leurs sels, à l'exception de ceux, où

X = oxygène et

a)

R = OH, $OCH_3$, $OC_2H_5$, $OCH_2CH=CH-C_6H_5$, $OCH_2CH=CH-CH_3$ ou $OCH_2CH_2CH=CH-CH_3$ et
n = 0, ainsi que

b)

R = un reste de formule

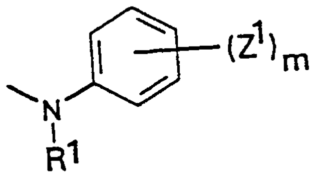

où $R^1$ = H et m = 0 ou $R^1$ = H et $(Z^1)_m$ = 4-Cl, et
n = 0, ainsi que

c)

R = $NH_2$ et
n = 0.

**10.** Procédé de préparation d'un composé selon la revendication 9, caractérisé en ce qu'on fait réagir un composé de formule II

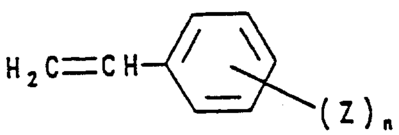

(II)

avec un composé de formule III

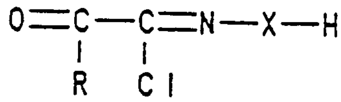

(III)

dans les formules II et III R, Z, n et X possèdent les définitions données à la formule I, et éventuellement on transforme le composé de formule I ainsi obtenu en son sel.

**11.** Procédé de lutte contre une végétation indésirable dans la culture de plantes utiles, caractérisé en ce qu'on applique au moins une substance active herbicide en combinaison avec au moins un composé de formule I défini selon les revendications 1 à 6, aux plantes, aux parties de plantes, à la semence ou à la surface cultivable.

**12.** Procédé de lutte contre une végétation indésirable dans la culture de plantes utiles, caractérisé en ce qu'on applique au moins une substance active herbicide en combinaison avec au moins un composé de formule I selon la revendication 10, aux plantes, aux parties de plantes, à la semence ou à la surface cultivable.

**13.** Utilisation d'un composé de formule I défini selon l'une des revendications 1 à 6 ou 9 pour la protection de cultures végétales contre les effets secondaires phytotoxiques des herbicides.

**14.** Procédé de préparation d'un agent selon l'une des revendications 1 à 8, caractérisé en ce qu'on met en forme d'application appropriée à la protection de végétaux au moins un composé, éventuellement ensemble avec une substance active herbicide, et des adjuvants de formulation.

**Revendications pour l'Etat contractant suivant : ES**

**1.** Utilisation de composés de formule I ou leurs sels

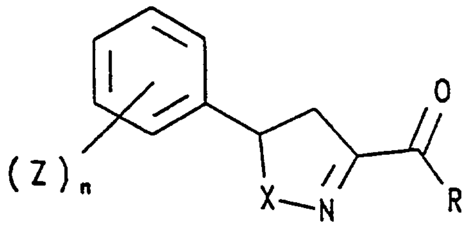

(I)

dans laquelle

X représente un atome d'oxygène ou de soufre,

R)

a) représente des groupes hydroxy, mercapto, alkoxy en $C_1$-$C_5$, alcényloxy en $C_2$-$C_5$, alcynyloxy en $C_2$-$C_5$, (alkyl en $C_1$-$C_5$)mercapto, (alcényl en $C_2$-$C_5$)mercapto, (alcynyl en $C_2$-$C_5$)mercapto, cycloalkoxy en $C_3$-$C_7$ ou (cycloalkyl en $C_3$-$C_7$)mercapto, les 8 derniers groupes pouvant être non substitués ou substitués par un ou plusieurs restes identiques ou différents pris dans le groupe des restes aryle en $C_6$-$C_{12}$, alkoxy en $C_1$-$C_5$, alcényloxy $C_2$-$C_5$, alcynyloxy en $C_2$-$C_5$, aralkyloxy en $C_6$-$C_{12}$, aryloxy en $C_6$-$C_{12}$, cycloalkoxy en $C_3$-$C_7$, (alkyl en $C_1$-$C_5$)mercapto, mono- ou di(alkyl en $C_1$-$C_5$)amino, cyano, halogène et nitro,

b) aralkyloxy en $C_6$-$C_{12}$, aryloxy en $C_6$-$C_{12}$, (aralkyl en $C_6$-$C_{12}$)mercapto ou (aryl en $C_6$-$C_{12}$)mercapto, chacun étant non substitué ou substitué par un ou plusieurs restes identiques ou différents pris dans le groupe des restes alkyle en $C_1$-$C_5$, alcényle en $C_2$-$C_5$, alcynyle en $C_2$-$C_5$, halogène, cyano, nitro, alkoxy en $C_1$-$C_5$, alcényloxy en $C_2$-$C_5$, alcynyloxy en $C_2$-$C_5$, (alkyl en $C_1$-$C_5$)mercapto, mono- ou di-(alkyl en $C_1$-$C_5$)amino, aryloxy en $C_6$-$C_{12}$ et aroyloxy en $C_6$-$C_{12}$, ou représente des groupes tri(alkyl en $C_1$-$C_5$)-silyl-(alkoxy en $C_1$-$C_5$), (aryl en $C_6$-$C_{12}$)-di-(alkyl en $C_1$-$C_5$)silyloxy, (aryl en $C_6$-$C_{12}$)-(alkyl en $C_1$-$C_5$)-di-(alkyl en $C_1$-$C_5$)silyloxy, di-(aryl en $C_6$-$C_{12}$)-(alkyl en $C_1$-$C_5$)silyloxy ou di-[(aryl en $C_6$-$C_{12}$)-(alkyl en $C_1$-$C_5$)]-(alkyl en $C_1$-$C_5$)silyloxy,

c) un reste de formule -NR'R', où R' représente des restes identiques ou différents pris dans le groupe comportant des atomes d'hydrogène, des groupes alkyle en $C_1$-$C_5$, alcényle en $C_2$-$C_5$, alcynyle en $C_2$-$C_5$ et cycloalkyle en $C_3$-$C_7$, ou des groupes pyridino, morpholino, di-(alkyl en $C_1$-$C_5$)morpholino, hydrazino ou un reste de formule

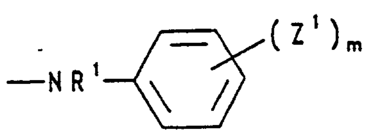

où $R^1$ représente un atome d'hydrogène, des groupes alkyle en $C_1$-$C_5$, alcényle en $C_2$-$C_5$ ou alcynyle en $C_2$-$C_5$, les restes $Z^1$ représentent, indépendamment les uns des autres, des atomes d'halogènes, des groupes nitro, alkyle en $C_1$-$C_5$, alcényle en $C_2$-$C_5$, alkoxy en $C_1$-$C_5$ ou aryloxy en $C_6$-$C_{12}$ et m est un entier de 0 à 5,

d) un reste de formule

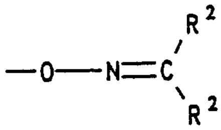

où les restes $R^2$ représentent indépendamment l'un de l'autre, des groupes alkyle en $C_1$-$C_5$ ou ensemble avec l'atome de carbone les reliant un groupe cycloalkylidène en $C_3$-$C_7$,

e) un reste de formule -O-$CR^3R^3$-CO-$R^4$, où $R^3$ représente des restes identiques ou différents pris dans le groupe comportant des atomes d'hydrogène, des groupes alkyle en $C_1$-$C_5$, alcényle en $C_2$-$C_5$, alcynyle en $C_2$-$C_5$, aryle en $C_6$-$C_{12}$, (aryl en $C_6$-$C_{12}$)-alkyle en $C_1$-$C_5$, alkoxy en $C_1$-$C_5$, alcényloxy en $C_2$-$C_5$, alcynyloxy en $C_2$-$C_5$ et aryloxy en $C_6$-$C_{12}$ et $R^4$ représente un atome d'hydrogène, des groupes alkyle en $C_1$-$C_5$, alcényle en $C_2$-$C_5$, alcynyle en $C_2$-$C_5$, aryle en $C_6$-$C_{12}$ ou aryl en $C_6$-$C_{12}$-alkyle en $C_1$-$C_5$,

f) un reste de formule

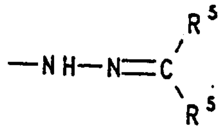

où les restes $R^5$ représentent des restes identiques ou différents pris dans le groupe comportant des atomes d'hydrogène, des restes alkyle en $C_1$-$C_5$ ou aryle en $C_6$-$C_{12}$ ou les deux restes $R^5$ ensemble avec l'atome de carbone les reliant, représentent un groupe cycloalkylidène en $C_3$-$C_7$, ou

g) un reste de formule -O-$CR^6R^6$-CO-$R^7$, où $R^6$ représente des restes identiques ou différents pris dans le groupe comportant des atomes d'hydrogène, des groupes alkyle en $C_1$-$C_5$, alcényle en $C_2$-$C_5$, alcynyle en $C_2$-$C_5$, aryle en $C_6$-$C_{12}$, (aryl en $C_6$-$C_{12}$)-alkyle en $C_1$-$C_5$, alkoxy en $C_1$-$C_5$, alcényloxy en $C_2$-$C_5$, alcynyloxy en $C_2$-$C_5$ et aryloxy en $C_6$-$C_{12}$ et $R^7$ possède l'une des significations données ci-dessus pour R aux points a) à f),

Z représente des atomes d'halogène, des groupes nitro, cyano, alkyle en $C_1$-$C_4$, alkoxy en $C_1$-$C_4$, (alkyl en $C_1$-$C_4$)mercapto, les groupes alkyle, alkoxy et alkylmercapto sont chacun, indépendamment l'un de l'autre, non substitués ou substitués par un ou plusieurs atomes d'halogène, un groupe cycloalkyle en $C_3$-$C_6$, lequel est non substitué ou substitué de préférence par jusqu'à trois groupes alkyle en $C_1$-$C_4$, représente des groupes amino, hydroxyméthyle, (alkyl en $C_1$-$C_4$)-amino, di-(alkyl en $C_1$-$C_4$)amino, (alkoxy en $C_1$-$C_4$)-méthyle, les groupes alkyle et alkoxy dans les trois derniers restes cités sont chacun, indépendamment les uns des autres, non substitué ou substitué par, de préférence, jusqu'à trois groupes alkyle en $C_1$-$C_4$, représente des groupes aryle en $C_6$-$C_{12}$ ou aryloxy en $C_6$-$C_{12}$, aryle et aryloxy étant chacun, indépendamment l'un de l'autre, non substitué ou substitué une ou plusieurs fois, de préférence jusqu'à 5 fois, par des restes identiques ou différents pris parmi les restes halogène et trifluorométhyle, et

n est un entier de 0 à 5, pour la protection de cultures végétales contre les effets secondaires phytotoxiques des herbicides.

**2.** Utilisation selon la revendication 1, où dans la formule I

R

a) représente des groupes hydroxy, mercapto, alkoxy en $C_1$-$C_4$, alcényloxy en $C_2$-$C_4$, alcynyloxy en $C_2$-$C_4$, (alkyl en $C_1$-$C_4$)mercapto, (alcényl en $C_2$-$C_4$)mercapto, (alcynyl en $C_2$-$C_4$)mercapto ou (cycloalkyl en $C_3$-$C_8$)-mercapto, les 8 derniers groupes pouvant être non substitués ou substitués par un ou plusieurs restes identiques ou différents pris dans le groupe des restes aryle, alkoxy en $C_1$-$C_4$, alcényloxy $C_2$-$C_4$, alcynyloxy en $C_2$-$C_4$, aralkyloxy, aryloxy, cycloalkoxy en $C_3$-$C_8$, (alkyl en $C_1$-$C_4$)mercapto, mono- ou di(alkyl en $C_1$-$C_4$)-amino, cyano, halogène et nitro,

b) aryloxy, arylmercapto, aralkyloxy ou aralkylmercapto, chacun étant non substitué ou substitué par un ou plusieurs restes identiques ou différents pris dans le groupe des restes alkyle en $C_1$-$C_4$, alcényle en

$C_2$-$C_4$, alcynyle en $C_2$-$C_4$, halogène, cyano, nitro, alkoxy en $C_1$-$C_4$, alcényloxy en $C_2$-$C_4$, alcynyloxy en $C_2$-$C_4$, (alkyl en $C_1$-$C_4$)mercapto, mono- ou di-(alkyl en $C_1$-$C_4$)amino, aryloxy ou aralkyloxy, ou représente un groupe tri(alkyl en $C_1$-$C_4$)-silylalkoxy,

c) un reste de formule -NR'R', où R' représente un atome d'hydrogène et/ou un groupe alkyle en $C_1$-$C_4$, des groupes pyridino, morpholino, diméthylmorpholino, hydrazino ou un reste de formule

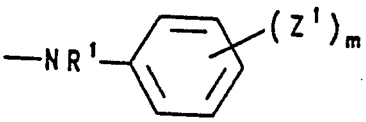

où $R^1$ représente un atome d'hydrogène ou un groupe alkyle en $C_1$-$C_4$, les restes $Z^1$ représentent, indépendamment les uns des autres, des atomes d'halogènes, des groupes nitro, alkyle en $C_1$-$C_4$, alkoxy en $C_1$-$C_4$ ou aryloxy et m est un entier de 0 à 3,

d) un reste de formule -O-N=$CR^2R^2$, où $R^2$ représente un groupe alkyle en $C_1$-$C_4$ ou les restes $R^2$ représentent ensemble avec l'atome de carbone qui les relie un groupe cyclohexylidène ou cyclopentylidène,

e) un reste de formule -O-$CR^3R^3$-CO-$R^4$, où $R^3$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, aryle, aralkyle ou alkoxy en $C_1$-$C_4$ et $R^4$ représente des groupes alkyle en $C_1$-$C_4$, alcényle en $C_2$-$C_4$, alcynyle en $C_2$-$c_4$, aryle ou aralkyle,

f) un reste de formule -NH-N=$CR^5R^5$, où $R^5$ représente des restes identiques ou différents pris dans le groupe comportant des atomes d'hydrogène, des groupes alkyle en $C_1$-$C_4$ ou aryle ou les deux restes $R^5$ représentent ensemble avec l'atome de carbone qui les relie un groupe cyclohexylidène ou cyclopentylidène, ou

g) un reste de formule -O-$CR^6R^6$-CO-$R^7$, où $R^6$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, aryle, aralkyle et alkoxy en $C_1$-$C_4$ et $R^7$ possède l'une des significations données pour R aux points a) à f).

**3.** Utilisation selon la revendication 1 ou 2, où dans la formule I

R

a) représente des groupes hydroxy, mercapto, alkoxy en $C_1$-$C_4$, alcényloxy en $C_2$-$C_4$, alcynyloxy en $C_2$-$C_4$, (alkyl en $C_1$-$C_4$)mercapto, (alcényl en $C_2$-$C_4$)mercapto, (alcynyl en $C_2$-$C_4$)mercapto ou (cycloalkyl en $C_3$-$C_8$)-mercapto, les 8 derniers groupes pouvant être non substitués ou substitués par jusqu'à trois restes identiques ou différents pris dans le groupe des restes phényle, alkoxy en $C_1$-$C_4$, alcényloxy $C_2$-$C_4$, alcynyloxy en $C_2$-$C_4$, benzyloxy, phényloxy, cycloalkoxy en $C_3$-$C_8$, (alkyl en $C_1$-$C_4$)mercapto, mono- ou di(alkyl en $C_1$-$C_4$)-amino, cyano, halogène et nitro,

b) phényloxy, phénylmercapto, benzyloxy ou benzylmercapto, chacun étant non substitué ou substitué par jusqu'à cinq restes identiques ou différents pris dans le groupe des restes alkyle en $C_1$-$C_4$, alcényle en $C_2$-$C_4$, alcynyle en $C_2$-$C_4$, halogène, cyano, nitro, alkoxy en $C_1$-$C_4$, alcényloxy en $C_2$-$C_4$, alcynyloxy en $C_2$-$C_4$, (alkyl en $C_1$-$C_4$)mercapto, mono- ou di-(alkyl en $C_1$-$C_4$)amino, phényloxy et benzyloxy, ou représente un groupe tri(alkyl en $C_1$-$C_4$)-silylalkoxy,

c) un reste de formule -NR'R', où R' représente un atome d'hydrogène et/ou un groupe alkyle en $C_1$-$C_4$, des groupes pyridino, morpholino, diméthylmorpholino, hydrazino ou un reste de formule

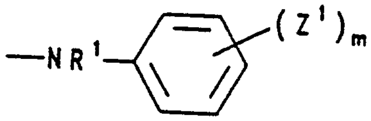

où $R^1$ représente un atome d'hydrogène ou un groupe alkyle en $C_1$-$C_4$, les restes $Z^1$ représentent, indépendamment les uns des autres, des atomes d'halogènes, des groupes nitro, alkyle en $C_1$-$C_4$, alkoxy en

$C_1$-$C_4$ ou phényloxy et m est un entier de 0 à 3,
d) un reste de formule -O-N=$CR^2R^2$, où $R^2$ représente un groupe alkyle en $C_1$-$C_4$ ou les restes $R^2$ représentent ensemble avec l'atome de carbone qui les relie un groupe cyclohexylidène ou cyclopentylidène,
e) un reste de formule -O-$CR^3R^3$-CO-$R^4$, où $R^3$ représente des restes identiques ou différents pris dans le groupe comportant des atomes d'hydrogène, des groupes alkyle en $C_1$-$C_4$, alcényle en $C_2$-$c_4$, alcynyle en $C_2$-$C_4$, phényle, benzyle ou alkoxy en $C_1$-$C_4$ et $R^4$ représente des groupes alkyle en $C_1$-$C_4$, alcényle en $C_2$-$C_4$, alcynyle en $C_2$-$C_4$, phényle ou benzyle,
f) un reste de formule -NH-N=$CR^5R^5$, où $R^5$ représente des restes identiques ou différents pris dans le groupe comportant des atomes d'hydrogène, des groupes alkyle en $C_1$-$C_4$ ou phényle ou les deux restes $R^5$ représentent ensemble avec l'atome de carbone qui les relie un groupe cyclohexylidène ou cyclopentylidène, ou
g) un reste de formule -O-$CR^6R^6$-CO-$R^7$, où $R^6$ représente des restes identiques ou différents pris dans le groupe comportant des atomes d'hydrogène, des groupes alkyle en $C_1$-$C_4$, alcényle en $C_2$-$C_4$, alcynyle en $C_2$-$C_4$, phényle, benzyle et alkoxy en $C_1$-$C_4$, et $R^7$ possède l'une des significations données pour R aux points a) à f).

**4.** Utilisation selon l'une des revendications 1 à 3, où dans la formule I

R représente un atome d'hydrogène, des groupes alkoxy en $C_1$-$C_4$, alcényloxy en $C_2$-$C_4$, alcynyloxy en $C_2$-$C_4$, , phényloxy, NR'R' avec R' = hydrogène et/ou alkyle en $C_1$-$C_4$, hydrazino ou -O-$CR^3R^3$-CO-$R^4$ avec $R^3$ = hydrogène et $R^4$ = alkoxy en $C_1$-$C_4$.

**5.** Utilisation selon l'une des revendications 1 à 4, où dans la formule I

Z représente des restes identiques ou différents pris dans le groupe comportant des atomes d'halogènes, des groupes alkyle en $C_1$-$C_4$ ou alkoxy en $C_1$-$C_4$ et
n vaut 0, 1 ou 2.

**6.** Utilisation selon l'une des revendications 1 à 5, où dans la formule I

X représente un atome d'oxygène.

**7.** Utilisation selon l'une des revendications 1 à 6, caractérisé en ce qu'il contient de plus au moins une substance active herbicide.

**8.** Utilisation selon la revendication 7, caractérisé en ce que la substance active herbicide est pris dans la classe des carbamates, des thiocarbamates, des halogénoacétanilides, des dérives substitués des acides phénoxy-, naphtoxy- et phénoxy-phénoxycarboxyliques, des dérivés d'acide hétéroaryloxyphénoxyalcane carboxylique, des dérivés de cyclohexane dione, des imidazolinones et des sulfonylurées.

**9.** Utilisation selon l'une des revendications 1 à 8, à l'exception de ceux, où dans les composés de formule (I)

X = oxygène et

a)

R = OH, $OCH_3$, $OC_2H_5$, $OCH_2CH$=CH-$C_6H_5$, $OCH_2CH$=CH-$CH_3$ ou $OCH_2CH_2CH$=CH-$CH_3$ et
n = 0, ainsi que

b)

R = un reste de formule

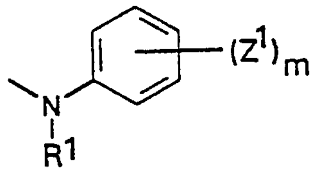

où $R^1$ = H et m = 0 ou $R^1$ = H et $(Z^1)_m$ = 4-Cl, et

n = 0, ainsi que

c)

R = $NH_2$ et

n = 0.

**10.** Procédé de préparation d'un composé selon la revendication 9, caractérisé en ce qu'on fait réagir un composé de formule II

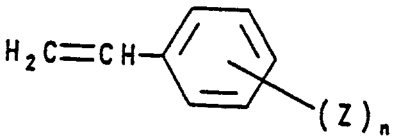

(II)

avec un composé de formule III

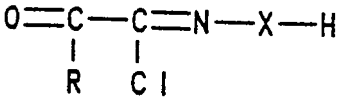

(III)

dans les formules II et III R, Z, n et X possèdent les définitions données à la formule I, et éventuellement on transforme le composé de formule I ainsi obtenu en son sel.

**11.** Procédé de lutte contre une végétation indésirable dans la culture de plantes utiles, caractérisé en ce qu'on applique au moins une substance active herbicide en combinaison avec au moins un composé de formule I défini selon les revendications 1 à 6, aux plantes, aux parties de plantes, à la semence ou à la surface cultivable.

**12.** Procédé de lutte contre une végétation indésirable dans la culture de plantes utiles, caractérisé en ce qu'on applique au moins une substance active herbicide en combinaison avec au moins un composé de formule I défini selon la revendication 10, aux plantes, aux parties de plantes, à la semence ou à la surface cultivable.

**13.** Procédé de préparation d'un agent phytoprotecteur caractérisé en ce qu'on met en forme d'application appropriée à la protection de végétaux au moins un composé de formule I ou ses sels selon l'une des revendications 1 à 8, éventuellement ensemble avec une substance active herbicide, et des adjuvants de formulation.